# EUROPEAN PATENT SPECIFICATION

(11) **EP 1 009 420 B1**
(45) Date of publication and mention of the grant of the patent: **17.12.2003**
(21) Application number: 98934444.5
(22) Date of filing: 09.07.1998
(51) Int. Cl.: A61K 38/03, A61K 38/07, A61K 38/08, C07K 5/00, C07K 7/00

(54) **CONJUGATES USEFUL IN THE TREATMENT OF PROSTATE CANCER**
KONJUGATE WELCHE BEI DER BEHANDLUNG VON PROSTATAKREBS NÜTZLICH SIND
CONJUGUES UTILES DANS LE TRAITEMENT DU CANCER DE LA PROSTATE

(30) Priority: 10.07.1997 US 52195 P; 13.05.1998 GB 9810183
(43) Date of publication of application: 21.06.2000
(73) Proprietor: MERCK & CO., INC., Rahway, New Jersey 07065 (US)
(72) Inventor: BRADY, Stephen, F., Rahway, NJ 07065 (US); GARSKY, Victor, M., Rahway, NJ 07065 (US); PAWLUCZYK, Joseph, M., Rahway, NJ 07065 (US)
(74) Representative: Thompson, John Dr.
(86) International application number: US9814413
(87) International publication number: WO99002175

(56) References cited:
- US-A- 5 502 037
- US-A- 5 599 686

### SEQUENCE LISTING

<110> Merck & Co., Inc.
   Brady, Stephen F.
   Garsky, Victor M.
   Pawluczyk, Joseph M.
<120> CONJUGATES USEFUL IN THE TREATMENT OF PROSTATE CANCER
<130> 19940Y
<150> 60/052,195
<151> 1997-07-10
<160> 103
<170> FastSEQ for Windows Version 3.0
<210> 1
<211> 7
<212> PRT
<213> Artificial Sequence
<400> 1
   Asn Lys Ile Ser Tyr Gln Ser
   1 5
<210> 2
<211> 6
<212> PRT
<213> Artificial Sequence
<400> 2
   Lys Ile Ser Tyr Gln Ser
   1 5
<210> 3
<211> 7
<212> PRT
<213> Artificial Sequence
<400> 3
   Asn Lys Ile Ser Tyr Tyr Ser
   1 5
<210> 4
<211> 7
<212> PRT
<213> Artificial Sequence
<400> 4
   Asn Lys Ala Ser Tyr Gln Ser
   1 5
<210> 5
<211> 5
<212> PRT
<213> Artificial Sequence
<400> 5
   Ser Tyr Gln Ser Ser
   1 5
<210> 6
<211> 5
<212> PRT
<213> Artificial Sequence
<400> 6
   Lys Tyr Gln Ser Ser
   1 5
<210> 7
<211> 5
<212> PRT
<213> Artificial Sequence
<220>
<221> VARIANT
<222> (1) ... (1)
<223> homoarginine
<400> 7
   Xaa Tyr Gln Ser Ser
   1 5
<210> 8
<211> 5
<212> PRT
<213> Artificial Sequence
<220>
<221> VARIANT
<222> (1) ... (1)
<223> homoarginine
<221> MOD_RES
<222> (0) ... (0)
<223> cyclohexylalanine
<400> 8
   Xaa Xaa Gln Ser Ser
   1 5
<210> 9
<211> 4
<212> PRT
<213> Artificial Sequence
<400> 9
   Tyr Gln Ser Ser
   1
<210> 10
<211> 4
<212> PRT
<213> Artificial Sequence
<400> 10
   Tyr Gln Ser Leu
   1
<210> 11
<211> 4
<212> PRT
<213> Artificial Sequence
<220>
<221> VARIANT
<222> (4) ... (4)
<223> norleucine
<400> 11
   Tyr Gln Ser Leu
   1
<210> 12
<211> 4
<212> PRT
<213> Artificial Sequence
<220>
<221> MOD_RES
<222> (0) ... (0)
<223> cyclohexylglycine
<400> 12
   Xaa Gln Ser Leu
   1
<210> 13
<211> 4
<212> PRT
<213> Artificial Sequence
<220>
<221> MOD_RES
<222> (1)...(1)
<223> cyclohexylglycine
<221> VARIANT
<222> (4) ... (4)
<223> norleucine
<400> 13
   Xaa Gln Ser Leu
   1
<210> 14
<211> 4
<212> PRT
<213> Artificial Sequence
<400> 14
   Ser Tyr Gln Ser
   1
<210> 15
<211> 4
<212> PRT
<213> Artificial Sequence
<220>
<221> MOD_RES
<222> (2) ... (2)
<223> cyclohexylglycine
<400> 15
   Ser Xaa Gln Ser
   1
<210> 16
<211> 5
<212> PRT
<213> Artificial Sequence
<400> 16
   Ser Tyr Gln Ser Val
   1 5
<210> 17
<211> 5
<212> PRT
<213> Artificial Sequence
<220>
<221> MOD_RES
<222> (2) ... (2)
<223> cyclohexylglycine
<400> 17
   Ser Xaa Gln Ser Val
   1 5
<210> 18
<211> 5
<212> PRT
<213> Artificial Sequence
<400> 18
   Ser Tyr Gln Ser Leu
   1 5
<210> 19
<211> 5
<212> PRT
<213> Artificial Sequence
<220>
<221> MOD_RES
<222> (2) ... (2)
<223> cyclohexylglycine
<400> 19
   Ser Xaa Gln Ser Leu
   1 5
<210> 20
<211> 6
<212> PRT
<213> Artificial Sequence
<220>
<221> MOD_RES
<222> (1) ... (1)
<223> cyclic amino acid substituted with a hydrophilic moiety
<400> 20
   Xaa Xaa Ser Tyr Gln Ser
   1 5
<210> 21
<211> 6
<212> PRT
<213> Artificial Sequence
<220>
<221> MOD_RES
<222> (1) ... (1)
<223> cyclic amino acid substituted with a hydrophilic moiety
<400> 21
   Xaa Xaa Lys Tyr Gln Ser
   1 5
<210> 22
<211> 6
<212> PRT
<213> Artificial Sequence
<220>
<221> MOD_RES
<222> (1) ... (1)
<223> cyclic amino acid substituted with a hydrophilic moiety
<221> VARIANT
<222> (3) ... (3)
<223> homoarginine
<400> 22
   Xaa Xaa Xaa Tyr Gln Ser
   1 5
<210> 23
<211> 6
<212> PRT
<213> Artificial Sequence
<220>
<221> MOD_RES
<222> (1) ... (1)
<223> cyclic amino acid substituted with a hydrophilic moiety
<221> VARIANT
<222> (3) ... (3)
<223> homoarginine
<221> MOD RES
<222> (4) ... (4)
<223> cyclohexylalanine
<400> 23
   Xaa Xaa Xaa Xaa Gln Ser
   1 5
<210> 24
<211> 4
<212> PRT
<213> Artificial Sequence
<220>
<221> MOD_RES
<222> (1) ... (1)
<223> cyclic amino acid substituted with a hydrophilic moiety
<400> 24
   Xaa Tyr Gln Ser
   1
<210> 25
<211> 6
<212> PRT
<213> Artificial Sequence
<220>
<221> MOD_RES
<222> (1) ... (1)
<223> cyclic amino acid substituted with a hydrophilic moiety
<221> MOD_RES
<222> (4) ... (4)
<223> cyclohexylglycine
<400> 25
   Xaa Xaa Ser Xaa Gln Ser
   1 5
<210> 26
<211> 4
<212> PRT
<213> Artificial Sequence
<220>
<221> MOD_RES
<222> (1) ... (1)
<223> cyclic amino acid substituted with a hydrophilic moiety
<221> MOD_RES
<222> (2) ... (2)
<223> cyclohexylglycine
<400> 26
   Xaa Xaa Gln Ser
   1
<210> 27
<211> 6
<212> PRT
<213> Artificial Sequence
<400> 27
   Ser Ser Tyr Gln Ser Val
   1 5
<210> 28
<211> 6
<212> PRT
<213> Artificial Sequence
<220>
<221> MOD_RES
<222> (3) ... (3)
<223> cyclohexylglycine
<400> 28
   Ser Ser Xaa Gln Ser Val
   1 5
<210> 29
<211> 6
<212> PRT
<213> Artificial Sequence
<400> 29
   Ser Ser Tyr Gln Ser Leu
   1 5
<210> 30
<211> 6
<212> PRT
<213> Artificial Sequence
<220>
<221> MOD_RES
<222> (3) ... (3)
<223> cyclohexylglycine
<400> 30
   Ser Ser Xaa Gln Ser Leu
   1 5
<210> 31
<211> 6
<212> PRT
<213> Artificial Sequence
<220>
<221> MOD_RES
<222> (1) ... (1)
<223> 4-hydroxyproline
<400> 31
   Pro Ser Ser Tyr Gln Ser
   1 5
<210> 32
<211> 6
<212> PRT
<213> Artificial Sequence
<220>
<221> MOD RES
<222> (1) ... (1)
<223> 4-hydroxyproline
<221> MOD_RES
<222> (4) ... (4)
<223> cyclohexylglycine
<400> 32
   Pro Ser Ser Xaa Gln Ser
   1 5
<210> 33
<211> 6
<212> PRT
<213> Artificial Sequence
<400> 33
   Ala Ser Tyr Gln Ser Val
   1 5
<210> 34
<211> 6
<212> PRT
<213> Artificial Sequence
<220>
<221> MOD_RES
<222> (3) ... (3)
<223> cyclohexylglycine
<400> 34
   Ala Ser Xaa Gln Ser Val
   1 5
<210> 35
<211> 6
<212> PRT
<213> Artificial Sequence
<400> 35
   Ala Ser Tyr Gln Ser Leu
   1 5
<210> 36
<211> 6
<212> PRT
<213> Artificial Sequence
<220>
<221> MOD_RES
<222> (3) ... (3)
<223> cyclohexylglycine
<400> 36
   Ala Ser Xaa Gln Ser Leu
   1 5
<210> 37
<211> 6
<212> PRT
<213> Artificial Sequence
<220>
<221> MOD_RES
<222> (1) ... (1)
<223> 4-hydroxyproline
<400> 37
   Pro Ala Ser Tyr Gln Ser
   1 5
<210> 38
<211> 6
<212> PRT
<213> Artificial Sequence
<220>
<221> MOD_RES
<222> (1) ... (1)
<223> 4-hydroxyproline
<221> MOD_RES
<222> (4) ... (4)
<223> cyclohexylglycine
<400> 38
   Pro Ala Ser Xaa Gln Ser
   1 5
<210> 39
<211> 6
<212> PRT
<213> Artificial Sequence
<220>
<221> MOD_RES
<222> (3) ... (3)
<223> cyclohexylglycine
<400> 39
   Ser Ser Xaa Gln Ser Leu
   1 5
<210> 40
<211> 6
<212> PRT
<213> Artificial Sequence
<220>
<221> MOD_RES
<222> (3) ... (3)
<223> cyclohexylglycine
<400> 40
   Ser Ser Xaa Gln Ser Val
   1 5
<210> 41
<211> 7
<212> PRT
<213> Artificial Sequence
<220>
<221> MOD_RES
<222> (4) ... (4)
<223> cyclohexylglycine
<400> 41
   Ser Ser Ser Xaa Gln Ser Leu
   1 5
<210> 42
<211> 7
<212> PRT
<213> Artificial Sequence
<220>
<221> MOD_RES
<222> (4) ... (4)
<223> cyclohexylglycine
<400> 42
   Ser Ser Ser Xaa Gln Ser Val
   1 5
<210> 43
<211> 7
<212> PRT
<213> Artificial Sequence
<220>
<221> MOD_RES
<222> (4) ... (4)
<223> cyclohexylglycine
<400> 43
   Ser Ala Ser Xaa Gln Ser Leu
   1 5
<210> 44
<211> 7
<212> PRT
<213> Artificial Sequence
<220>
<221> MOD RES
<222> (4) ... (4)
<223> cyclohexylglycine
<400> 44
   Ser Ala Ser Xaa Gln Ser Val
   1 5
<210> 45
<211> 7
<212> PRT
<213> Artificial Sequence
<220>
<221> METHYLATION
<222> (1) ... (1)
<223> N-methylserine
<221> MOD_RES
<222> (4) ... (4)
<223> cyclohexylglycine
<400> 45
   Xaa Ser Ser Xaa Gln Ser Leu
   1 5
<210> 46
<211> 7
<212> PRT
<213> Artificial Sequence
<220>
<221> METHYLATION
<222> (1) ... (1)
<223> N-methylserine
<221> MOD_RES
<222> (4) ... (4)
<223> cyclohexylglycine
<400> 46
   Xaa Ser Ser Xaa Gln Ser Val
   1 5
<210> 47
<211> 7
<212> PRT
<213> Artificial Sequence
<220>
<221> MOD RES
<222> (1) ... (1)
<223> 4-hydroxyproline
<400> 47
   Pro Ser Ser Tyr Gln Ser Val
   1 5
<210> 48
<211> 7
<212> PRT
<213> Artificial Sequence
<220>
<221> MOD_RES
<222> (1) ... (1)
<223> 4-hydroxyproline
<400> 48
   Pro Ser Ser Tyr Gln Ser Leu
   1 5
<210> 49
<211> 7
<212> PRT
<213> Artificial Sequence
<220>
<221> MOD_RES
<222> (1) ... (1)
<223> 4-hydroxyproline
<221> MOD_RES
<222> (4) ... (4)
<223> cyclohexylglycine
<400> 49
   Pro Ser Ser Xaa Gln Ser Val
   1 5
<210> 50
<211> 7
<212> PRT
<213> Artificial Sequence
<220>
<221> MOD_RES
<222> (1) ... (1)
<223> 4-hydroxyproline
<221> MOD_RES
<222> (4) ... (4)
<223> cyclohexylglycine
<400> 50
   Pro Ser Ser Xaa Gln Ser Leu
   1 5
<210> 51
<211> 7
<212> PRT
<213> Artificial Sequence
<220>
<221> MOD_RES
<222> (1) ... (1)
<223> 4-hydroxyproline
<221> MOD_RES
<222> (4) ... (4)
<223> cyclohexylglycine
<400> 51
   Pro Ala Ser Xaa Gln Ser Val
   1 5
<210> 52
<211> 7
<212> PRT
<213> Artificial Sequence
<220>
<221> MOD_RES
<222> (1) ... (1)
<223> 4-hydroxyproline
<221> MOD_RES
<222> (4) ... (4)
<223> cyclohexylglycine
<400> 52
   Pro Ala Ser Xaa Gln Ser Leu
   1 5
<210> 53
<211> 7
<212> PRT
<213> Artificial Sequence
<220>
<221> MOD_RES
<222> (1) ... (1)
<223> 3,4-dihydroxyproline
<400> 53
   Xaa Ser Ser Tyr Gln Ser Val
   1 5
<210> 54
<211> 7
<212> PRT
<213> Artificial Sequence
<220>
<221> MOD_RES
<222> (1) ... (1)
<223> 3,4-dihydroxyproline
<400> 54
   Xaa Ser Ser Tyr Gln Ser Val
   1 5
<210> 55
<211> 7
<212> PRT
<213> Artificial Sequence
<220>
<221> VARIANT
<222> (1) ... (1)
<223> homoarginine
<221> MOD_RES
<222> (4) ... (4)
<223> cyclohexylglycine
<400> 55
   Xaa Ser Ala Xaa Gln Ser Leu
   1 5
<210> 56
<211> 7
<212> PRT
<213> Artificial Sequence
<220>
<221> VARIANT
<222> (1) ... (1)
<223> homoarginine
<221> MOD_RES
<222> (3) ... (3)
<223> 4-hydroxyproline
<221> MOD_RES
<222> (4) ... (4)
<223> cyclohexylglycine
<400> 56
   Xaa Ser Pro Xaa Gln Ser Leu
   1 5
<210> 57
<211> 5
<212> PRT
<213> Artificial Sequence
<220>
<221> MOD_RES
<222> (1) ... (1)
<223> 4-hydroxyproline
<221> MOD RES
<222> (2) ... (2)
<223> cyclohexylglycine
<400> 57
   Pro Xaa Gln Ser Leu
   1 5
<210> 58
<211> 7
<212> PRT
<213> Artificial Sequence
<400> 58
   Asn Arg Ile Ser Tyr Gln Ser
   1 5
<210> 59
<211> 7
<212> PRT
<213> Artificial Sequence
<400> 59
   Asn Lys Val Ser Tyr Gln Ser
   1 5
<210> 60
<211> 8
<212> PRT
<213> Artificial Sequence
<400> 60
   Asn Lys Met Ser Tyr Gln Ser Ser
   1 5
<210> 61
<211> 8
<212> PRT
<213> Artificial Sequence
<400> 61
   Asn Lys Leu Ser Tyr Gln Ser Ser
   1 5
<210> 62
<211> 7
<212> PRT
<213> Artificial Sequence
<400> 62
   Asn Lys Ile Ser Tyr Gln Ser
   1 5
<210> 63
<211> 8
<212> PRT
<213> Artificial Sequence
<400> 63
   Gln Lys Ile Ser Tyr Gln Ser Ser
   1 5
<210> 64
<211> 7
<212> PRT
<213> Artificial Sequence
<220>
<221> MOD_RES
<222> (2) ... (2)
<223> 4-hydroxyproline
<400> 64
   Asn Pro Ile Ser Tyr Gln Ser
   1 5
<210> 65
<211> 7
<212> PRT
<213> Artificial Sequence
<220>
<221> MOD_RES
<222> (1) ... (1)
<223> 4-hydroxyproline
<400> 65
   Asn Pro Val Ser Tyr Gln Ser
   1 5
<210> 66
<211> 7
<212> PRT
<213> Artificial Sequence
<220>
<221> MOD_RES
<222> (1) ... (1)
<223> 4-hydroxyproline
<400> 66
   Pro Ala Ser Tyr Gln Ser Ser
   1 5
<210> 67
<211> 7
<212> PRT
<213> Artificial Sequence
<220>
<221> MOD_RES
<222> (1) ... (1)
<223> 3,4-dihydroxyproline
<400> 67
   Xaa Ala Ser Tyr Gln Ser Ser
   1 5
<210> 68
<211> 5
<212> PRT
<213> Artificial Sequence
<220>
<221> MOD_RES
<222> (1) ... (1)
<223> 3-hydroxyproline
<221> MOD_RES
<222> (3) ... (3)
<223> cyclohexylglycine
<400> 68
   Pro Ser Xaa Gln Ser
   1 5
<210> 69
<211> 7
<212> PRT
<213> Artificial Sequence
<220>
<221> MOD_RES
<222> (1) ... (1)
<223> 4-hydroxyproline
<221> MOD_RES
<222> (4) ... (4)
<223> cyclohexylglycine
<400> 69
   Pro Ala Ser Xaa Gln Ser Ser
   1 5
<210> 70
<211> 7
<212> PRT
<213> Artificial Sequence
<220>
<221> MOD_RES
<222> (1) ... (1)
<223> N-(PEG-2)-4-hydroxyproline
<221> MOD_RES
<222> (4) ... (4)
<223> cyclohexylglycine
<400> 70
   Xaa Ser Ser Xaa Gln Ser Val
   1 5
<210> 71
<211> 7
<212> PRT
<213> Artificial Sequence
<220>
<221> ACETYLATION
<222> (1) ... (1)
<223> N-acetylserine
<221> MOD_RES
<222> (4) ... (4)
<223> cyclohexylglycine
<400> 71
   Xaa Ser Ser Xaa Gln Ser Leu
   1 5
<210> 72
<211> 7
<212> PRT
<213> Artificial Sequence
<220>
<221> MOD_RES
<222> (1) ... (1)
<223> N-(PEG-2)-4-hydroxyproline
<221> MOD_RES
<222> (4) ... (4)
<223> cyclohexylglycine
<400> 72
   Xaa Ser Ser Xaa Gln Ser Leu
   1 5
<210> 73
<211> 7
<212> PRT
<213> Artificial Sequence
<220>
<221> ACETYLATION
<222> (1) ... (1)
<223> N-acetylserine
<221> MOD_RES
<222> (4) ... (4)
<223> cyclohexylglycine
<400> 73
   Xaa Ser Ser Xaa Gln Ser Val
   1 5
<210> 74
<211> 7
<212> PRT
<213> Artificial Sequence
<220>
<221> ACETYLATION
<222> (1) ... (1)
<223> N-acetylproline
<221> MOD_RES
<222> (4) ... (4)
<223> cyclohexylglycine
<400> 74
   Xaa Ser Ser Xaa Gln Ser Val
   1 5
<210> 75
<211> 7
<212> PRT
<213> Artificial Sequence
<220>
<221> ACETYLATION
<222> (1) ... (1)
<223> N-acetylserine
<221> MOD_RES
<222> (4) ... (4)
<223> cyclohexylglycine
<221> VARIANT
<222> (7) ... (7)
<223> valine having the unnatural configuration
<400> 75
   Xaa Ser Ser Xaa Gln Ser Xaa
   1 5
<210> 76
<211> 6
<212> PRT
<213> Artificial Sequence
<220>
<221> ACETYLATION
<222> (1) ... (1)
<223> N-acetylserine
<221> MOD_RES
<222> (4) ... (4)
<223> cyclohexylglycine
<400> 76
   Xaa Ser Ser Xaa Gln Ser
   1 5
<210> 77
<211> 7
<212> PRT
<213> Artificial Sequence
<220>
<221> MOD_RES
<222> (1) ... (1)
<223> N-hydroxyacetyl-4-hydroxyproline
<221> MOD_RES
<222> (4) ... (4)
<223> cyclohexylglycine
<400> 77
   Xaa Ser Ser Xaa Gln Ser Val
   1 5
<210> 78
<211> 7
<212> PRT
<213> Artificial Sequence
<220>
<221> MOD_RES
<222> (1) ... (1)
<223> N-(PEG-2)-4-hydroxyproline
<221> MOD_RES
<222> (4) ... (4)
<223> cyclohexylglycine
<400> 78
   Xaa Ser Ser Xaa Gln Ser Val
   1 5
<210> 79
<211> 7
<212> PRT
<213> Artificial Sequence
<220>
<221> MOD_RES
<222> (1) ... (1)
<223> N-(PEG-2)-3,4-dihydroxyproline
<221> MOD_RES
<222> (4) ... (4)
<223> cyclohexylglycine
<400> 79
   Xaa Ser Ser Xaa Gln Ser Val
   1 5
<210> 80
<211> 7
<212> PRT
<213> Artificial Sequence
<220>
<221> MOD_RES
<222> (1) ... (1)
<223> N-acetyl-3,4-dihydroxyproline
<221> MOD_RES
<222> (4) ... (4)
<223> cyclohexylglycine
<221> VARIANT
<222> (7)...(7)
<223> 2-amino-4,4-dimethylpropanoic acid
<400> 80
   Xaa Ser Ser Xaa Gln Ser Val
   1 5
<210> 81
<211> 7
<212> PRT
<213> Artificial Sequence
<220>
<221> MOD_RES
<222> (1) ... (1)
<223> N-succinyl-trans-4-hydroxyproline
<221> MOD_RES
<222> (4) ... (4)
<223> cyclohexylglycine
<400> 81
   Xaa Ser Ser Xaa Gln Ser Val
   1 5
<210> 82
<211> 7
<212> PRT
<213> Artificial Sequence
<220>
<221> METHYLATION
<222> (1) ... (1)
<223> N-(PEG-2)-N-methylserine
<221> MOD_RES
<222> (4) ... (4)
<223> cyclohexylglycine
<400> 82
   Xaa Ser Ser Xaa Gln Ser Val
   1 5
<210> 83
<211> 7
<212> PRT
<213> Artificial Sequence
<220>
<221> METHYLATION
<222> (1) ... (1)
<223> N-methylserine
<221> MOD_RES
<222> (4) ... (4)
<223> cyclohexylglycine
<400> 83
   Xaa Ser Ser Xaa Gln Ser Val
   1 5
<210> 84
<211> 7
<212> PRT
<213> Artificial Sequence
<220>
<221> BLOCKED
<222> (1) ... (1)
<223> N-quinylserine
<221> MOD_RES
<222> (4) ... (4)
<223> cyclohexylglycine
<400> 84
   Xaa Ser Ser Xaa Gln Ser Val
   1 5
<210> 85
<211> 7
<212> PRT
<213> Artificial Sequence
<220>
<221> BLOCKED
<222> (1) ... (1)
<223> N-gallylserine
<221> MOD_RES
<222> (4) ... (4)
<223> cyclohexylglycine
<400> 85
   Xaa Ser Ser Xaa Gln Ser Val
   1 5
<210> 86
<211> 7
<212> PRT
<213> Artificial Sequence
<220>
<221> MOD_RES
<222> (1) ... (1)
<223> N-(PEG-2)-trans-4-hydroxyproline
<221> MOD_RES
<222> (4) ... (4)
<223> cyclohexylglycine
<400> 86
   Xaa Ala Ser Xaa Gln Ser Val
   1 5
<210> 87
<211> 7
<212> PRT
<213> Artificial Sequence
<220>
<221> MOD_RES
<222> (1) ... (1)
<223> N-(4-imidazolylacetyl)-trans-4-hydroxyproline
<221> MOD_RES
<222> (4) ... (4)
<223> cyclohexylglycine
<400> 87
   Xaa Ser Ser Xaa Gln Ser Val
   1 5
<210> 88
<211> 7
<212> PRT
<213> Artificial Sequence
<220>
<221> ACETYLATION
<222> (1) ... (1)
<223> N-acetyl-histidine
<221> MOD_RES
<222> (4) ... (4)
<223> cyclohexylglycine
<400> 88
   Xaa Ala Ser Xaa Gln Ser Val
   1 5
<210> 89
<211> 7
<212> PRT
<213> Artificial Sequence
<220>
<221> MOD_RES
<222> (1) ... (1)
<223> N-(PEG-2)-cysteic acid
<221> MOD_RES
<222> (4) ... (4)
<223> cyclohexylglycine
<400> 89
   Xaa Ser Ser Xaa Gln Ser Val
   1 5
<210> 90
<211> 7
<212> PRT
<213> Artificial Sequence
<220>
<221> MOD_RES
<222> (1) ... (1)
<223> N-gulonic-trans-4-hydroxyproline
<221> MOD_RES
<222> (4) ... (4)
<223> cyclohexylglycine
<400> 90
   Xaa Ser Ser Xaa Gln Ser Val
   1 5
<210> 91
<211> 7
<212> PRT
<213> Artificial Sequence
<220>
<221> MOD_RES
<222> (1) ... (1)
<223> N-(4-phosphonylbutylryl)-trans-4-hydroxyproline
<221> MOD_RES
<222> (4) ... (4)
<223> cyclohexylglycine
<400> 91
   Xaa Ser Ser Xaa Gln Ser Val
   1 5
<210> 92
<211> 7
<212> PRT
<213> Artificial Sequence
<220>
<221> MOD_RES
<222> (1) ... (1)
<223> N-cotinyl-trans-4-hydroxyproline
<221> MOD_RES
<222> (4) ... (4)
<223> cyclohexylglycine
<400> 92
   Xaa Ser Ser Xaa Gln Ser Val
   1 5
<210> 93
<211> 7
<212> PRT
<213> Artificial Sequence
<220>
<221> MOD_RES
<222> (1) ... (1)
<223> N-(3-phosphonylpropionyl)-trans-4-hydroxyproline
<221> MOD_RES
<222> (4) ... (4)
<223> cyclohexylglycine
<400> 93
   Xaa Ser Ser Xaa Gln Ser Val
    1               5
<210> 94
<211> 7
<212> PRT
<213> Artificial Sequence
<220>
<221> MOD_RES
<222> (1) ... (1)
<223> N-succinyl-trans-4-hydroxyproline
<221> MOD_RES
<222> (4) ... (4)
<223> cyclohexylglycine
<400> 94
   Xaa Ser Ser Xaa Gln Ser Val
    1               5
<210> 95
<211> 7
<212> PRT
<213> Artificial Sequence
<220>
<221> MOD RES
<222> (1) ... (1)
<223> N-glutaryl-trans-4-hydroxyproline
<221> MOD_RES
<222> (4) ... (4)
<223> cyclohexylglycine
<400> 95
   Xaa Ser Ser Xaa Gln Ser Val
    1               5
<210> 96
<211> 7
<212> PRT
<213> Artificial Sequence
<220>
<221> MOD_RES
<222> (1) ... (1)
<223> N-ethoxysquarate-trans-4-hydroxyproline
<221> MOD_RES
<222> (4) ... (4)
<223> cyclohexylglycine
<400> 96
   Xaa Ser Ser Xaa Gln Ser Val
   1 5
<210> 97
<211> 7
<212> PRT
<213> Artificial Sequence
<220>
<221> MOD RES
<222> (1) ... (1)
<223> N-(PEG-2)-trans-4-hydroxyproline
<221> MOD_RES
<222> (4) ... (4)
<223> cyclohexylglycine
<400> 97
   Xaa Ser Ser Xaa Gln Ser Leu
   1 5
<210> 98
<211> 7
<212> PRT
<213> Artificial Sequence
<220>
<221> ACETYLATION
<222> (1) ... (1)
<223> N-acetylserine
<221> MOD_RES
<222> (4) ... (4)
<223> cyclohexylglycine
<400> 98
   Xaa Ser Ser Xaa Gln Ser Leu
   1 5
<210> 99
<211> 7
<212> PRT
<213> Artificial Sequence
<220>
<221> ACETYLATION
<222> (1) ... (1)
<223> N-acetylserine
<221> MOD_RES
<222> (4) ... (4)
<223> cyclohexylglycine
<400> 99
   Xaa Ser Ser Xaa Gln Ser Leu
   1 5
<210> 100
<211> 7
<212> PRT
<213> Artificial Sequence
<220>
<221> MOD_RES
<222> (1) ... (1)
<223> N-glutaryl-trans-4-hydroxyproline
<221> MOD_RES
<222> (4) ... (4)
<223> cyclohexylglycine
<400> 100
   Xaa Ala Ser Xaa Gln Ser Leu
   1 5
<210> 101
<211> 5
<212> PRT
<213> Artificial Sequence
<220>
<221> ACETYLATION
<222> (1) ... (1)
<223> N-acetylserine
<221> MOD_RES
<222> (4) ... (4)
<223> cyclohexylglycine
<400> 101
   Xaa Xaa Gln Ser Leu
   1 5
<210> 102
<211> 6
<212> PRT
<213> Artificial Sequence
<220>
<221> ACETYLATION
<222> (1) ... (1)
<223> N-acetylserine
<221> MOD_RES
<222> (3) ... (3)
<223> cyclohexylglycine
<400> 102
   Xaa Ser Xaa Gln Ser Leu
   1 5
<210> 103
<211> 7
<212> PRT
<213> Artificial Sequence
<220>
<221> MOD_RES
<222> (1) ... (1)
<223> N-acetyl-beta-keto-4-hydroxyproline
<221> MOD_RES
<222> (4) ... (4)
<223> cyclohexylglycine
<400> 103
   Xaa Ala Ser Xaa Gln Ser Leu
    1               5

## Description

### BACKGROUND OF THE INVENTION

In 1996 cancer of the prostate gland was expected to be diagnosed in 317,000 men in the U.S. and 42,000 American males die from this disease (Garnick, M.B. (1994). The Dilemmas of Prostate Cancer. Scientific American, April:72-81). Thus, prostate cancer is the most frequently diagnosed malignancy (other than that of the skin) in U.S. men and the second leading cause of cancer-related deaths (behind lung cancer) in that group.

Prostate specific Antigen (PSA) is a single chain 33 kDa glycoprotein that is produced almost exclusively by the human prostate epithelium and occurs at levels of 0.5 to 2.0 mg/ml in human seminal fluid (Nadji, M., Taber, S.Z., Castro, A., et al. (1981) Cancer 48:1229; Papsidero, L., Kuriyama, M., Wang, M., et al. (1981). JNCI 66:37; Qui, S.D., Young, C.Y.F., Bihartz, D.L., et al. (1990), J. Urol. 144:1550; Wang, M.C., Valenzuela, L.A., Murphy, G.P., et al. (1979). Invest. Urol. 17:159). The single carbohydrate unit is attached at asparagine residue number 45 and accounts for 2 to 3 kDa of the total molecular mass. PSA is a protease with chymotrypsin-like specificity (Christensson, A., Laurell, C.B., Lilja, H. (1990). Eur. J. Biochem. 194:755-763). It has been shown that PSA is mainly responsible for dissolution of the gel structure formed at ejaculation by proteolysis of the major proteins in the sperm entrapping gel, Semenogelin I and Semenogelin II, and fibronectin (Lilja, H. (1985). J. Clin. Invest. 76:1899; Lilja, H., Oldbring, J., Rarinevik, G., et al. (1987). J. Clin. Invest. 80:281; McGee, R.S., Herr, J.C. (1988). Biol. Reprod. 39:499). The PSA mediated proteolysis of the gel-forming proteins generates several soluble Semenogelin I and Semenogelin II fragments and soluble fibronectin fragments with liquefaction of the ejaculate and release of progressively motile spermatoza (Lilja, H., Laurell, C.B. (1984). Scand. J. Clin. Lab. Invest. 44:447; McGee, R.S., Herr, J.C. (1987). Biol. Reprod. 37:431). Furthermore, PSA may proteolytically degrade IGFBP-3 (insulin-like growth factor binding protein 3) allowing IGF to stimulate specifically the growth of PSA secreting cells (Cohen et al., (1992) J. Clin. Endo. & Meta. 75:1046-1053).

PSA complexed to alpha 1 - antichymotrypsin is the predominant molecular form of serum PSA and may account for up to 95% of the detected serum PSA (Christensson, A., Björk, T., Nilsson, O., et al. (1993). J. Urol. 150:100-105; Lilja, H., Christensson, A., Dahlén, U. (1991). Clin. Chem. 37:1618-1625; Stenman, U.H., Leinoven, J., Alfthan, H., et al. (1991). Cancer Res. 51:222-226). The prostatic tissue (normal, benign hyperplastic, or malignant tissue) is implicated to predominantly release the mature, enzymatically active form of PSA, as this form is required for complex formation with alpha 1 - antichymotrypsin (Mast, A.E., Enghild, J.J., Pizzo, S.V., et al. (1991). Biochemistry 30:1723-1730; Perlmutter, D.H., Glover, G.I., Rivetna, M., et al. (1990). Proc. Natl. Acad. Sci. USA 87:3753-3757). Therefore,.in the microenvironment of prostatic PSA secreting cells the PSA is believed to be processed and secreted in its mature enzymatically active form not complexed to any inhibitory molecule. PSA also forms stable complexes with alpha 2-macroglobulin, but as this results in encapsulation of PSA and complete loss of the PSA epitopes, the in vivo significance of this complex formation is unclear. A free, noncomplexed form of PSA constitutes a minor fraction of the serum PSA (Christensson, A., Björk, T., Nilsson, O., et al. (1993). J. Urol. 150:100-105; Lilja, H., Christensson, A., Dahlén, U. (1991). Clin. Chem. 37:1618-1625). The size of this form of serum PSA is similar to that of PSA in seminal fluid (Lilja, H., Christensson, A., Dahlén, U. (1991). Clin. Chem. 37:1618-1625) but it is yet unknown as to whether the free form of serum PSA may be a zymogen; an internally cleaved, inactive form of mature PSA; or PSA manifesting enzyme activity. However, it seems unlikely that the free form of serum PSA manifests enzyme activity, since there is considerable (100 to 1000 fold) molar excess of both unreacted alpha 1 - antichymotrypsin and alpha 2 - macroglobulin in serum as compared with the detected serum levels of the free 33 kDa form of PSA (Christensson, A., Björk, T., Nilsson, O., et al. (1993). J. Urol. 150:100-105; Lilja, H., Christensson, A., Dahlén, U. (1991). Clin. Chem. 37:1618-1625).

Serum measurements of PSA are useful for monitoring the treatment of adenocarcinoma of the prostate (Duffy, M.S. ( 1989). Ann. Clin. Biochem. 26:379-387; Brawer, M.K. and Lange, P.H. (1989). Urol. Suppl. 5:11-16; Hara, M. and Kimura, H. (1989). J. Lab. Clin. Med. 113:541-548), although above normal serum concentrations of PSA have also been reported in benign prostatic hyperplasia and subsequent to surgical trauma of the prostate (Lilja, H., Christensson, A., Dahlén, U. (1991). Clin. Chem. 37:1618-1625). Prostate metastases are also known to secrete immunologically reactive PSA since serum PSA is detectable at high levels in prostatectomized patients showing widespread metatstatic prostate cancer (Ford, T.F., Butcher, D.N., Masters, R.W., et al. (1985). Brit. J. Urology 57:50-55). Therefore, a cytotoxic compound that could be activated by the proteolytic activity of PSA should be prostate cell specific as well as specific for PSA secreting prostate metastases.

See also US 5,502,037 and 5,599, 686 which disclose conjugates with linear linkers.

It is the object of this invention to provide a novel anti-cancer composition useful for the treatment of prostate cancer which comprises oligopeptides, that are selectively proteolytically cleaved by free prostate specific antigen (PSA) and that include a cyclic amino acid having a hydrophilic substituent, in conjugation with a cytotoxic agent.

Another object of this invention is to provide a method of treating prostate cancer which comprises administration of the novel anti-cancer composition.

### SUMMARY OF THE INVENTION

Chemical conjugates which comprise oligopeptides, having amino acid sequences that are selectively proteolytically cleaved by free prostate specific antigen (PSA), and a cytotoxic agent are disclosed. The conjugates of the invention are characterized by a diamine linker comprising a cyclic alkyl moiety between the oligopeptide and vinblastine. Such conjugates are useful in the treatment of prostatic cancer and benign prostatic hyperplasia (BPH).

### DETAILED DESCRIPTION OF THE INVENTION

The instant invention relates to novel anti-cancer compositions useful for the treatment of prostate cancer. Such compositions comprise the oligopeptides covalently bonded through a chemical linker to a vinca drug. The oligopeptides are chosen from oligomers that are selectively recognized by the free prostate specific antigen (PSA) and are capable of being proteolytically cleaved by the enzymatic activity of the free prostate specific antigen. Such a combination of an oligopeptide and cytotoxic agent may be termed a conjugate.

The conjugates of the instant invention are characterized by a linker between the C-terminus of the oligopeptide and the vinca drug. The linker is a diamine comprising a cyclic alkyl moiety and most preferably, the diamine comprises a bicycloalkyl moiety. Examples of such diamine linkers include but are not limited to 1,4-bis(aminomethyl)cyclohexane, 1,4-bis(aminomethyl)cycloheptane, 1,3-bis(aminomethyl)cyclopentane, 1-amino-4-(aminomethyl) cyclohexane, 1,4-diaminocyclohexane, 1,4-bis(aminomethyl)bicyclo [2.2.2]octane.

Ideally, the cytotoxic activity of vinblastine is greatly reduced or absent when the oligopeptide containing the PSA proteolytic cleavage site is bonded through a chemical linker to the cytotoxic agent and is intact. Also ideally, the cytotoxic activity of the cytotoxic agent increases significantly or returns to the activity of the unmodified cytotoxic agent upon proteolytic cleavage of the attached oligopeptide at the cleavage site.

Furthermore, it is preferred that the oligopeptide is selected from oligopeptides that are not cleaved or are cleaved at a much slower rate in the presence of non-PSA proteolytic enzymes, such as those enzymes endogenous to human serum, when compared to the cleavage of the oligopeptides in the presence of free enzymatically active PSA.

For the reasons above, it is desireable for the oligopeptide to comprise a short peptide sequence, preferably less than ten amino acids. Most preferably the oligopeptide comprises seven or six amino acids. Because the conjugate preferably comprises a short amino acid sequence, the solubility of the conjugate may be influenced to a greater extent by the generally hydrophobic character of the cytotoxic agent component. Therefore, amino acids with hydrophilic substituents may be incorporated in the oligopeptide sequence or N-terminus blocking groups may be selected to offset or diminish such a hydrophobic contribution by the cytotoxic agent.

While it is not necessary for practicing this aspect of the invention, a preferred embodiment of this invention is a conjugate wherein the oligopeptide and the chemical linker are detached from the cytotoxic agent by the proteolytic activity of the free PSA and any other native proteolytic enzymes present in the tissue proximity, thereby presenting the cytotoxic agent, or a cytotoxic agent that retains part of the oligopeptide/linker unit but remains cytotoxic, into the physiological environment at the place of proteolytic cleavage. Pharmaceutically acceptable salts of the conjugates are also included.

It is understood that the oligopeptide that is conjugated to the cytotoxic agent does not need to be the oligopeptide that has the greatest recognition by free PSA and is most readily proteolytically cleaved by free PSA. Thus, the oligopeptide that is selected for incorporation in such an anti-cancer composition will be chosen both for its selective, proteolytic cleavage by free PSA and for the cytotoxic activity of the cytotoxic agent-proteolytic residue conjugate (or, in what is felt to be an ideal situation, the unmodified cytotoxic agent) which results from such a cleavage. The term "selective" as used in connection with the proteolytic PSA cleavage means a greater rate of cleavage of an oligopeptide component of the instant invention by free PSA relative to cleavage of an oligopeptide which comprises a random sequence of amino acids. Therefore, the oligopeptide component of the instant invention is a prefered substrate of free PSA. The term "selective" also indicates that the oligopeptide is proteolytically cleaved by free PSA between two specific amino acids in the oligopeptide.

The oligopeptide components of the instant invention are selectively recognized by the free prostate specific antigen (PSA) and are capable of being proteolytically cleaved by the enzymatic activity of the free prostate specific antigen. Such oligopeptides comprise an oligomer selected from:
a)
b)
c)
d)
e)
f)
g)
h)
i)
j)
k)
l)
m)
n)
o)
p)
q)
r)
s)
t)
u)
v)
w)
x)
y)
z)
wherein Haa is a cyclic amino acid substituted with a hydrophilic moiety, hArg is homoarginine, Xaa is any amino acid, Cha is cyclohexylalanine and Chg is cyclohexylglycine.

In an embodiment of the instant invention, the oligopeptide comprises an oligomer that is selected from:
a)
b)
c)
d)
e)
f)
h)
i)
j)
k)
l)
m)
wherein 4-Hyp is 4-hydroxyproline, Xaa is any amino acid, hArg is homoarginine, Cha is cyclohexylalanine and Chg is cyclohexylglycine.

In a more preferred embodiment of the instant invention, the oligopeptide comprises an oligomer selected from: and wherein 4-Hyp is 4-hydroxyproline, 3,4-DiHyp is 3,4-dihydroxyproline and Chg is cyclohexylglycine.

The phrase "oligomers that comprise an amino acid sequence" as used hereinabove, and elsewhere in the Detailed Description of the Invention, describes oligomers of from about 3 to about 100 amino acids residues which include in their amino acid sequence the specific amino acid sequence decribed and which are therefore proteolytically cleaved within the amino acid sequence described by free PSA. Preferably, the oligomer is from 5 to 10 amino acid residues. Thus, for example, the following oligomer: comprises the amino acid sequence: and would therefore come within the instant invention. And the oligomer: comprises the amino acid sequence: and would therefore come within the instant invention. It is understood that such oligomers do not include semenogelin I, semenogelin II, fibronectin and IGFBP-3.

A person of ordinary skill in the peptide chemistry art would readily appreciate that certain amino acids in a biologically active oligopeptide may be replaced by other homologous, isosteric and/or isoelectronic amino acids wherein the biological activity of the original oligopeptide has been conserved in the modified oligopeptide. Certain unnatural and modified natural amino acids may also be utilized to replace the corresponding natural amino acid in the oligopeptides of the instant invention. Thus, for example, tyrosine may be replaced by 3-iodotyrosine, 2-methyltyrosine, 3-fluorotyrosine, 3-methyltyrosine and the like. Further for example, lysine may be replaced with N'-(2-imidazolyl)lysine and the like. The following list of amino acid replacements is meant to be illustrative and is not limiting:

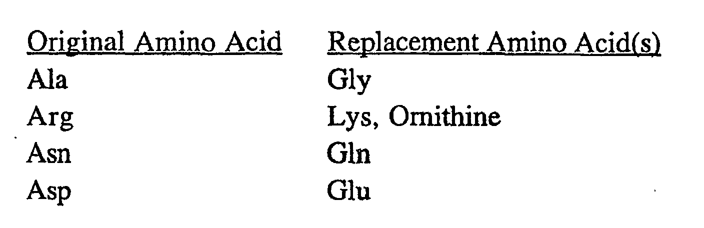

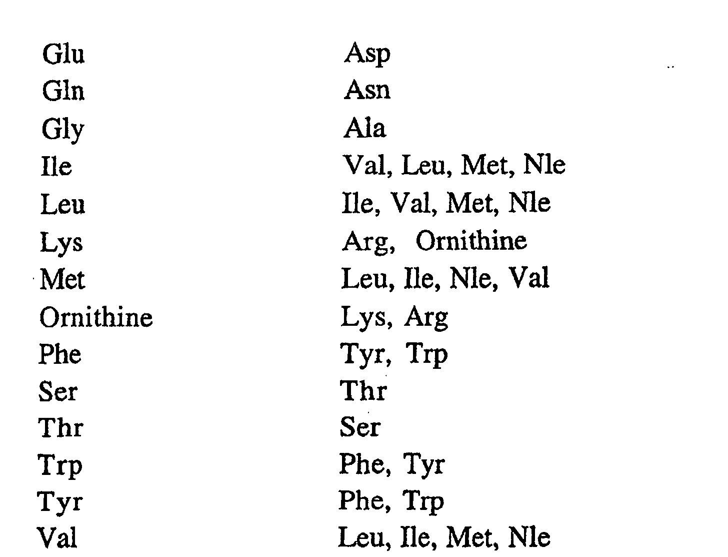

Thus, for example, the following oligopeptides may be synthesized by techniques well known to persons of ordinary skill in the art and would be expected to be proteolytically cleaved by free PSA:

The inclusion of the symbol "I" within an amino acid sequence indicates the point within that sequence where the oligopeptide is proteolytically cleaved by free PSA.

The compounds of the present invention may have asymmetric centers and occur as racemates, racemic mixtures, and as individual diastereomers, with all possible isomers, including optical isomers, being included in the present invention. Unless otherwise specified, named amino acids are understood to have the natural "L" stereoconfiguration

In the present invention, the amino acids which are disclosed are identified both by conventional 3 letter and single letter abbreviations as indicated below:

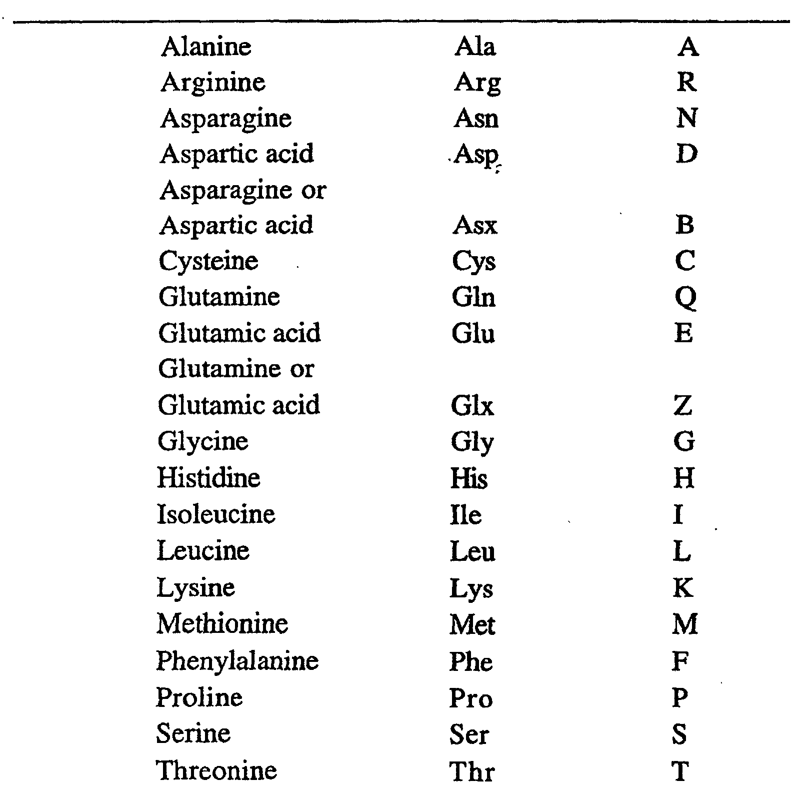

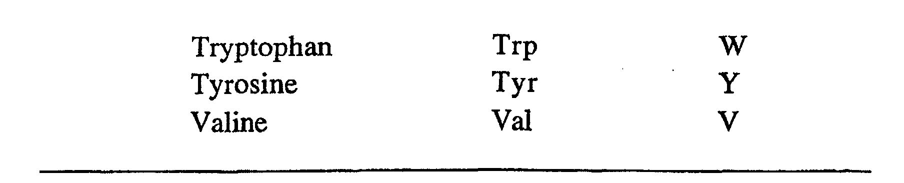

The following abbreviations are utilized in the specification and figures to denote the indicated amino acids and moieties:

| | |
|---|---|
| hR or hArg | homoarginine |
| hY or hTyr | homotyrosine |
| Cha | cyclohexylalanine |
| Amf | 4-aminomethylphenylalanine |
| DAP | 1,3-diaminopropyl |
| DPL | 2-(4,6-dimethylpyrimidinyl)lysine |
| (imidazolyl)K | N'-(2-imidazolyl)lysine |
| Me₂PO₃-Y | O-dimethylphosphotyrosine |
| O-Me-Y | O-methyltyrosine |
| TIC | 1,2,3,4-tetrahydro-3-isoquinoline carboxylic acid |
| DAP | 1,3-diaminopropane |
| TFA | trifluoroacetic acid |
| AA | acetic acid |
| 3PAL | 3-pyridylalanine |
| 4-Hyp | 4-hydroxyproline |
| dAc-Vin | 4-*des*- acetylvinblastine |

It is well known in the art, and understood in the instant invention, that peptidyl therapeutic agents such as the instant oligopeptide-cytotoxic agent conjugates preferably have the terminal amino moiety of any oligopeptide substituent protected with a suitable protecting group, such as acetyl, benzoyl, pivaloyl and the like. Such protection of the terminal amino group reduces or eliminates the enzymatic degradation of such peptidyl therapeutic agents by the action of exogenous amino peptidases which are present in the blood plasma of warm blooded animals. Such protecting groups also include hydrophilic blocking groups, which are chosen based upon the presence of hydrophilic functionality. Blocking groups that increase the hydrophilicity of the conjugates and therefore increase the aqueous solubility of the conjugates include but are not limited to hydroylated alkanoyl, polyhydroxylated alkanoyl, polyethylene glycol, glycosylates, sugars and crown ethers. N-Terminus unnatural amino acid moieties may also ameleorate such enzymatic degradation by exogenous amino peptidases.

Preferably the N-terminus protecting group is selected from
a) acetyl:
b) 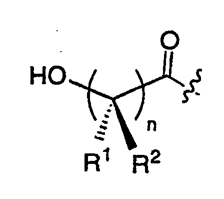
c) 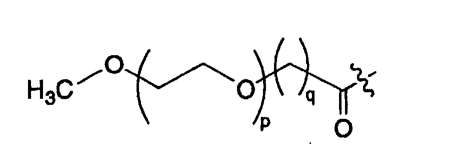
d) 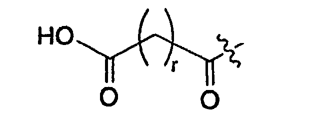
wherein:
R¹ and R² are independently selected from:
   a) hydrogen,
   b) unsubstituted or substituted aryl, unsubstituted or substituted heterocycle, C₃-C₁₀ cycloalkyl, C₂-C₆ alkenyl, C₂-C₆ alkynyl, halogen, C₁-C₆ perfluoroalkyl, R³O-, R³C(O)NR³-, (R³)₂NC(O)-, R³₂N-C(NR³)-, R⁴S(O)₂NH, CN, NO₂, R³C(O)-, N₃, -N(R³)₂, or R⁴OC(O)NR³-,
   c) unsubstituted C₁-C₆ alkyl,
   d) substituted C₁-C₆ alkyl wherein the substituent on the substituted C₁-C₆ alkyl is selected from unsubstituted or substituted aryl, unsubstituted or substituted heterocyclic, C₃-C₁₀ cycloalkyl, C₂-C₆ alkenyl, C₂-C₆ alkynyl, R³O-, R⁴S(O)₂NH, R³C(O)NR³-, (R³)₂NC(O)-, R³₂N-C(NR³)-, CN, R³C(O)-, N₃, -N(R³)₂, and R⁴OC(O)-NR³-; or
R¹ and R² are combined to form - (CH₂)ₛ - wherein one of the carbon atoms is optionally replaced by a moiety selected from: O, S(O)ₘ, -NC(O)-, NH and -N(COR⁴)- ;
R³ is selected from: hydrogen, aryl, substituted aryl, heterocycle, substituted heterocycle, C₁-C₆ alkyl and C₃-C₁₀ cycloalkyl;
R⁴ is selected from: aryl, substituted aryl, heterocycle, substituted heterocycle, C₁-C₆ alkyl and C₃-C₁₀ cycloalkyl;

- m: is 0, 1 or 2;
- n: is 1, 2, 3 or 4;
- p: is zero or an integer between 1 and 100; and
- q: is 0 or 1, provided that if p is zero, q is 1; and
- r: is 1, 2 or 3;
- s: is 3, 4 or 5.

Certain of the oligopeptides of the instant conjugates comprise a cyclic amino acid substituted with a hydrophilic moiety, previously represented by the term "Haa", which may also be represented by the formula: 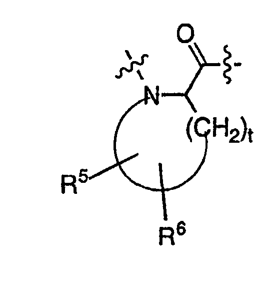 wherein:
R⁵ is selected from HO- and C₁-C₆ alkoxy;
R⁶ is selected from hydrogen, halogen, C₁-C₆ alkyl, HO- and C₁-C₆ alkoxy; and
t is 3 or 4.
The structure 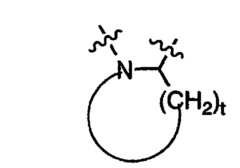 represents a cyclic amine moiety having 5 or 6 members in the ring, such a cyclic amine which may be optionally fused to a phenyl or cyclohexyl ring. Examples of such a cyclic amine moiety include, but are not limited to, the following specific structures:

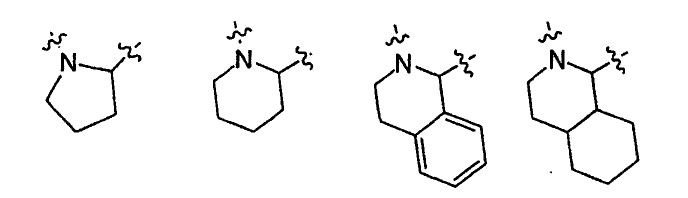

The conjugates of the present invention may have asymmetric centers and occur as racemates, racemic mixtures, and as individual diastereomers, with all possible isomers, including optical isomers, being included in the present invention. When any variable (e.g. aryl, heterocycle, R³ etc.) occurs more than one time in any constituent, its definition on each occurence is independent of every other occurence. For example, HO(CR¹R²)₂- represents HOCH₂CH₂-, HOCH₂CH(OH)-, HOCH(CH₃)CH(OH)-, etc. Also, combinations of substituents and/or variables are permissible only if such combinations result in stable compounds.

As used herein, "alkyl" and the alkyl portion of aralkyl and similar terms, is intended to include both branched and straight-chain saturated aliphatic hydrocarbon groups having the specified number of carbon atoms; "alkoxy" represents an alkyl group of indicated number of carbon atoms attached through an oxygen bridge.

As used herein, "cycloalkyl" is intended to include nonaromatic cyclic hydrocarbon groups having the specified number of carbon atoms. Examples of cycloalkyl groups include cyclopropyl, cyclobutyl, cyclopentyl, cyclohexyl and the like.

"Alkenyl" groups include those groups having the specified number of carbon atoms and having one or several double bonds. Examples of alkenyl groups include vinyl, allyl, isopropenyl, pentenyl, hexenyl, heptenyl, cyclopropenyl, cyclobutenyl, cyclopentenyl, cyclohexenyl, 1-propenyl, 2-butenyl, 2-methyl-2-butenyl, isoprenyl, farnesyl, geranyl, geranylgeranyl and the like.

"Alkynyl" groups include those groups having the specified number of carbon atoms and having one triple bonds. Examples of alkynyl groups include acetylene, 2-butynyl, 2-pentynyl, 3-pentynyl and the like.

"Halogen" or "halo" as used herein means fluoro, chloro, bromo and iodo.

As used herein, "aryl," and the aryl portion of aralkyl and aroyl, is intended to mean any stable monocyclic or bicyclic carbon ring of up to 7 members in each ring, wherein at least one ring is aromatic. Examples of such aryl elements include phenyl, naphthyl, tetrahydronaphthyl, indanyl, biphenyl, phenanthryl, anthryl or acenaphthyl.

The term heterocycle or heterocyclic, as used herein, represents a stable 5- to 7-membered monocyclic or stable 8- to 11-membered bicyclic heterocyclic ring which is either saturated or unsaturated, and which consists of carbon atoms and from one to four heteroatoms selected from the group consisting of N, O, and S, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include, but are not limited to, azepinyl, benzimidazolyl, benzisoxazolyl, benzofurazanyl, benzopyranyl, benzothiopyranyl, benzofuryl, benzothiazolyl, benzothienyl, benzoxazolyl, chromanyl, cinnolinyl, dihydrobenzofuryl, dihydrobenzothienyl, dihydrobenzothiopyranyl, dihydrobenzothiopyranyl sulfone, furyl, imidazolidinyl, imidazolinyl, imidazolyl, indolinyl, indolyl, isochromanyl, isoindolinyl, isoquinolinyl, isothiazolidinyl, isothiazolyl, isothiazolidinyl, morpholinyl, naphthyridinyl, oxadiazolyl, 2-oxoazepinyl, oxazolyl, 2-oxopiperazinyl, 2-oxopiperdinyl, 2-oxopyrrolidinyl, piperidyl, piperazinyl, pyridyl, pyrazinyl, pyrazolidinyl, pyrazolyl, pyridazinyl, pyrimidinyl, pyrrolidinyl, pyrrolyl, quinazolinyl, quinolinyl, quinoxalinyl, tetrahydrofuryl, tetrahydroisoquinolinyl, tetrahydroquinolinyl, thiamorpholinyl, thiamorpholinyl sulfoxide, thiazolyl, thiazolinyl, thienofuryl, thienothienyl, and thienyl.

As used herein in the terms "substituted C₁₋₈ alkyl", "substituted aryl" and "substituted heterocycle" include moieties containing from 1 to 3 substituents in addition to the point of attachment to the rest of the compound. Such additional substituents are selected from F, Cl, Br, CF₃, NH₂, N(C₁-C₆ alkyl)₂, NO₂, CN, (C₁-C₆ alkyl)O-, -OH, (C₁-C₆ alkyl)S(O)ₘ-, (C₁-C₆ alkyl)C(O)NH-, H₂N-C(NH)-, (C₁-C₆ alkyl)C(O)-, (C₁-C₆ alkyl)OC(O)-, N₃, (C₁-C₆ alkyl)OC(O)NH- and C₁-C₂₀ alkyl.

When R¹ and R² are combined to form - (CH₂)s -, the cyclic moieties and heteroatom-containing cyclic moieties so defined include, but are not limited to:

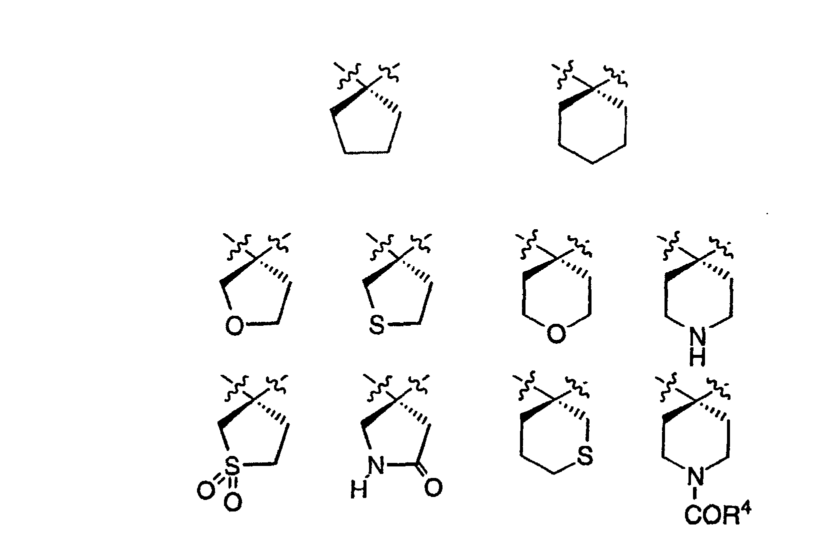

As used herein, the term "hydroxylated" represents substitution on a substitutable carbon of the ring system being so described by a hydroxyl moiety. As used herein, the term "polyhydroxylated" represents substitution on two or more substitutable carbon of the ring system being so described by 2, 3 or 4 hydroxyl moieties.

As used herein, the term "PEG" represents certain polyethylene glycol containing substituents having the designated number of ethyleneoxy subunits. Thus the term PEG(2) represents 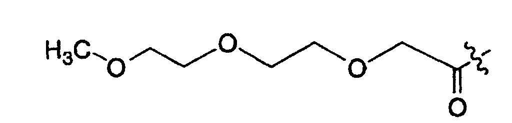 and the term PEG(6) represents

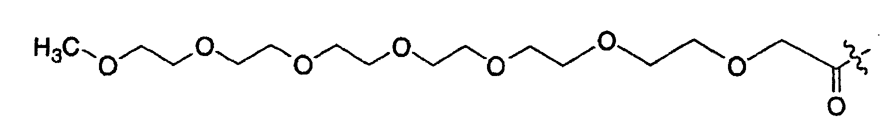

As used herein, the term "(d)(2,3-dihydroxypropionyl)" represents the following structure:

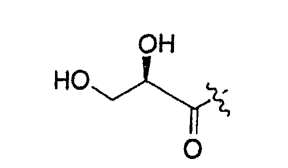

As used herein, the term "(2R,3S) 2,3,4-trihydroxybutanoyl" represents the following structure:

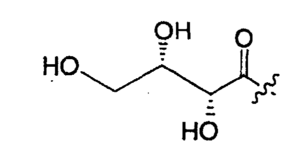

As used herein, the term "quinyl" represents the following structure: 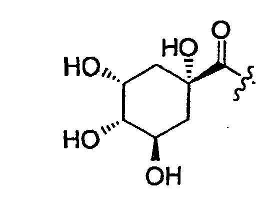 or the diastereomer thereof.

As used herein, the term "gulonic" represents the following structure: 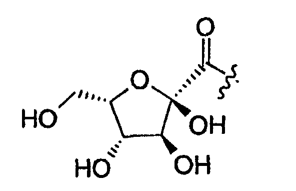 or the diastereomer thereof.

As used herein, the term "cotininyl" represents the following structure: 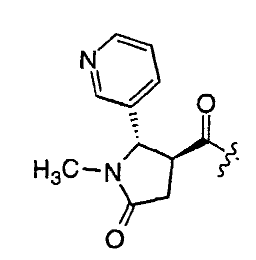 or the diastereomer thereof.

As used herein, the term "gallyl" represents the following structure:

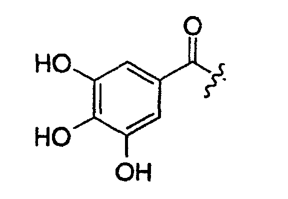

As used herein, the term "4-ethoxysquarate" represents the following structure:

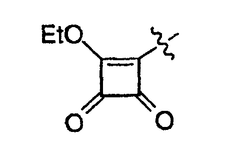

As used herein, the terms "BDAM" and "1,4-BDAM" both represent bis-1,4-aminomethylbicyclo-[2.2.2]octane:

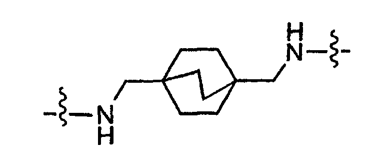

The cytotoxic agent that is utilized in the conjugates of the instant invention are the vinca alkaloid cytotoxic agents. Particularly useful members of this class include, for example, vinblastine, desacetylvinblastine, vincristine, leurosidine, vindesine, vinorelbine, navelbine, leurosine and the like. One skilled in the art may make chemical modifications to the desired cytotoxic agent in order to make reactions of that compound more convenient for purposes of preparing conjugates of the invention.

The preferred group of cytotoxic agents for the present invention include drugs of the following formulae:

### THE VINCA ALKALOID GROUP OF DRUGS OF FORMULA (1):

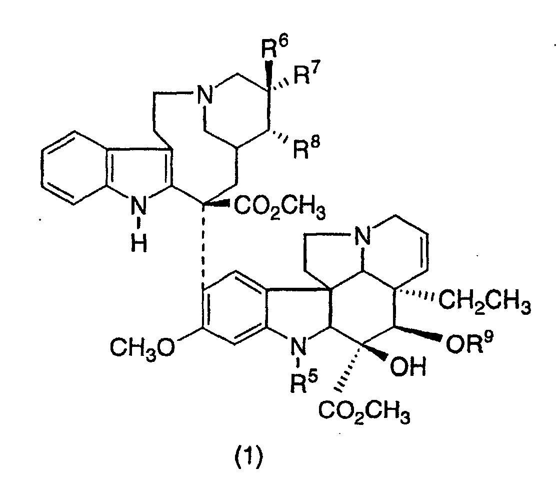

in which
R⁵ is H, CH₃ or CHO;
when R⁷ and R⁸ are taken singly, R⁸ is H, and one of R¹⁶ and R¹⁷ is ethyl and the other is H or OH;
when R⁷ and R⁸ are taken together with the carbons to which they are attached, they form an oxirane ring in which case R⁶ is ethyl;
R⁹ is hydrogen, (C₁-C₃ alkyl)-CO, or chlorosubstituted (C₁-C₃ alkyl)-CO.

The oligopeptide-cytotoxic agent conjugate of the instant invention wherein the cytotoxic agent is the preferred cytotoxic agent vinblastine may be described by the general formula I below: 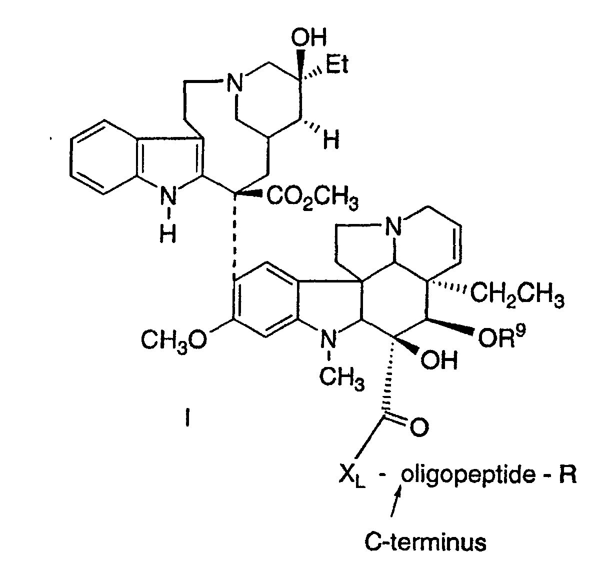 wherein:
oligopeptide is an oligopeptide which is specifically recognized by the free prostate specific antigen (PSA) and is capable of being proteolytically cleaved by the enzymatic activity of the free prostate specific antigen,
X_{L} is -NH-(CH₂)ᵤ-W-(CH₂)ᵤ-NH-
R is selected from
   a) hydrogen,
   b) -(C=O)R^{1a},
   c) 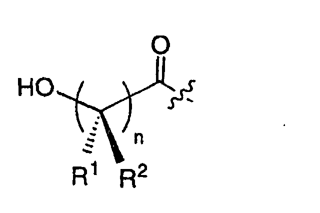
   d) 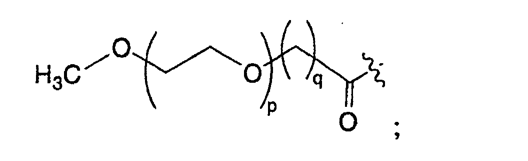
   e) 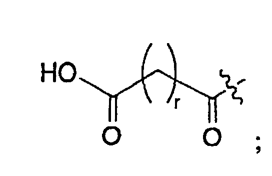
   f) ethoxysquarate; and
   g) cotininyl;
R¹ and R² are independently selected from: hydrogen, OH, C₁-C₆ alkyl, C₁-C₆ alkoxy, C₁-C₆ aralkyl and aryl;
R^{1a} is C₁-C₆-alkyl, hydroxylated C₃-C₈-cycloalkyl, polyhydroxylated C₃-C₈-cycloalkyl, hydroxylated aryl, polyhydroxylated aryl or aryl,
R⁹ is hydrogen, (C₁-C₃ alkyl)-CO, or chlorosubstituted (C₁-C₃ alkyl)-CO;
W is selected from cyclopentyl, cyclohexyl, cycloheptyl or bicyclo[2.2.2]octanyl;

- n is: 1, 2, 3 or 4;
- p is: zero or an integer between 1 and 100;
- q is: 0 or 1, provided that if p is zero, q is 1;
- ris: 1, 2 or 3;
- t is: 3 or 4;
- u is: 0, 1, 2 or 3,
or the pharmaceutically acceptable salt thereof.

The following compounds are specific examples of the oligopeptide-desacetylvinblastine conjugate of the instant invention: 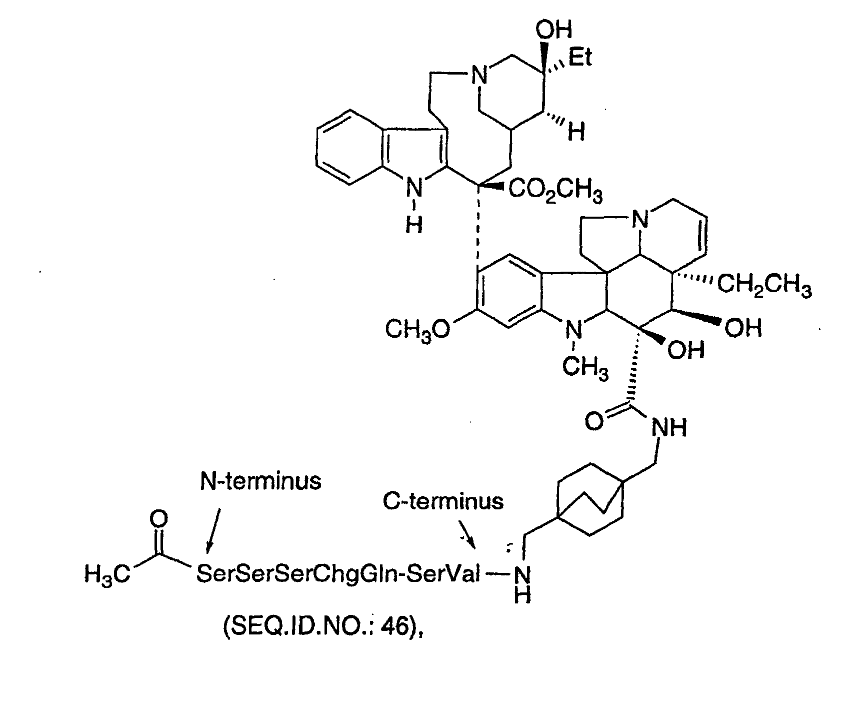 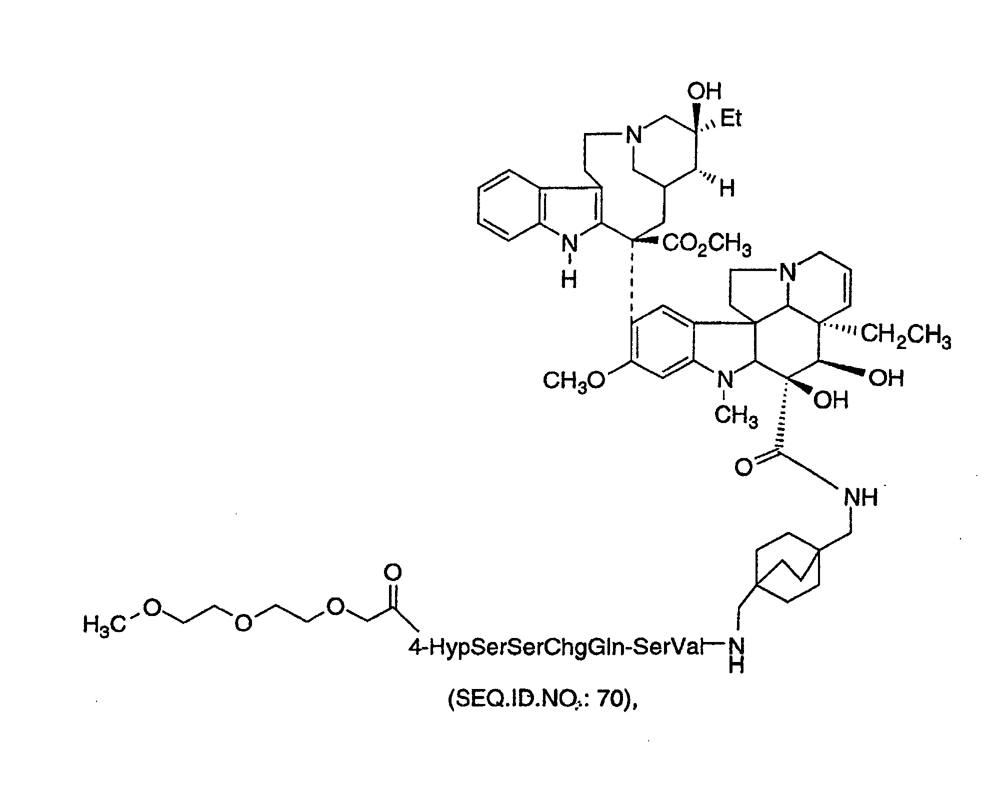 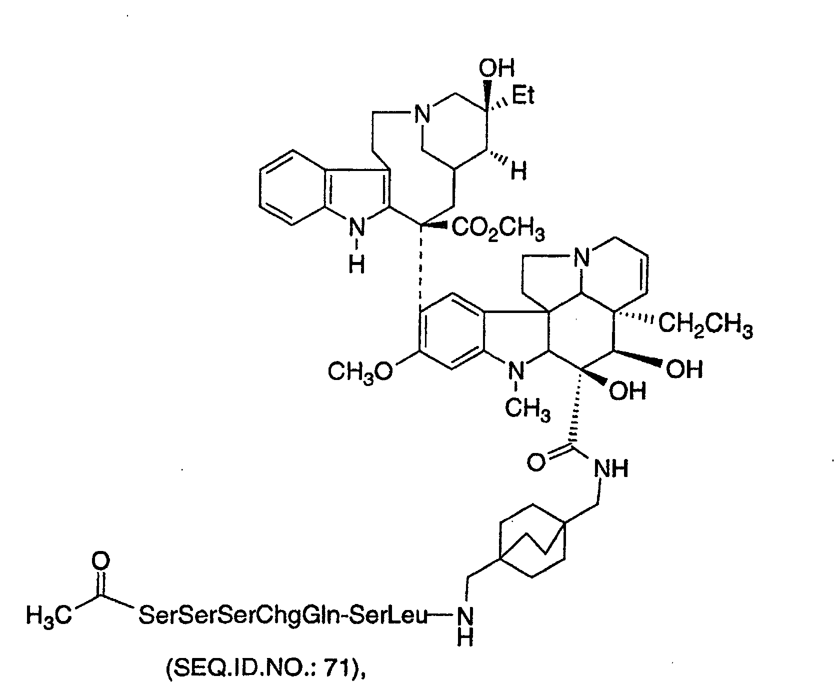 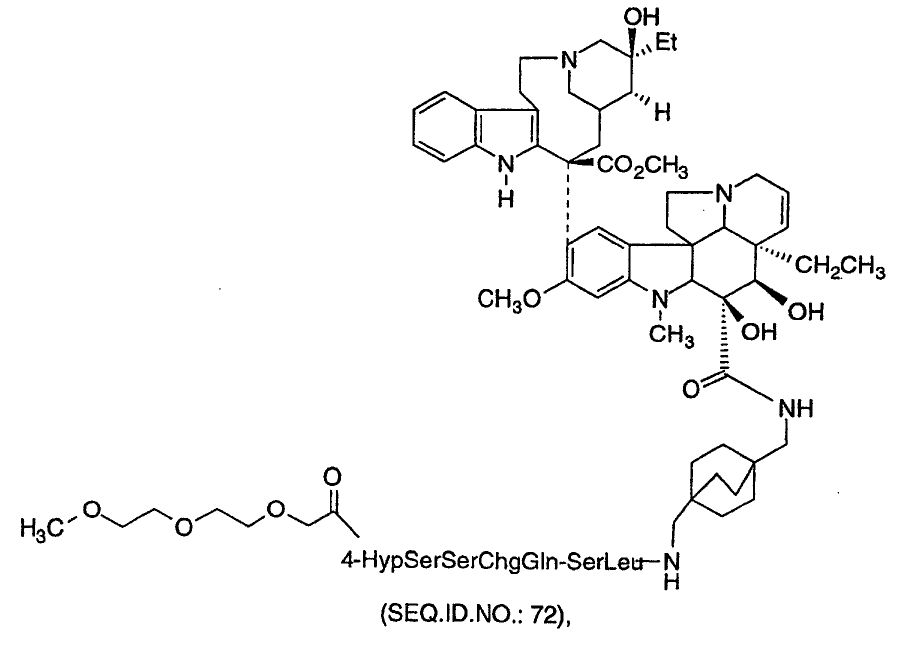 or the pharmaceutically acceptable salt thereof.

The oligopeptides, peptide subunits and peptide derivatives (also termed "peptides") of the present invention can be synthesized from their constituent amino acids by conventional peptide synthesis techniques, preferably by solid-phase technology. The peptides are then purified by reverse-phase high performance liquid chromatography (HPLC).

Standard methods of peptide synthesis are disclosed, for example, in the following works: Schroeder *et al*., "The Peptides", Vol. I, Academic Press 1965; Bodansky *et al*., "Peptide Synthesis", Interscience Publishers, 1966; McOmie (ed.) "Protective Groups in Organic Chemistry", Plenum Press, 1973; Barany *et al*., "The Peptides: Analysis, Synthesis, Biology" 2, Chapter 1, Academic Press, 1980, and Stewart *et al*., *"Solid Phase Peptide Synthesis"*, Second Edition, Pierce Chemical Company, 1984.

The suitably substituted cyclic amino acid having a hydrophilic substituent, which may be incorporated into the instant conjugates by standard peptide synthesis techniques, is itself either commercially available or is readily synthesized by techniques well known in the art or described herein. Thus syntheses of suitably substituted prolines are described in the following articles and references cited therein: J. Ezquerra et al., *J. Org. Chem*. 60: 2925-2930 (1995); P. Gill and W. D. Lubell, *J. Org. Chem*., 60:2658-2659 (1995); and M. W. Holladay et al., *J. Med. Chem*., 34:457-461 (1991).

The pharmaceutically acceptable salts of the compounds of this invention include the conventional non-toxic salts of the compounds of this invention as formed, e.g., from non-toxic inorganic or organic acids. For example, such conventional non-toxic salts include those derived from inorganic acids such as hydrochloric, hydrobromic, sulfuric, sulfamic, phosphoric, nitric and the like: and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, trifluoroacetic and the like.

The conjugates of the instant invention which comprise the oligopeptide containing the PSA cleavage site, the cyclic linker, and a cytotoxic agent may similarly be synthesized by techniques well known in the medicinal chemistry art. For these purposes a reagent such as 2-(1H-benzotriazol-1-yl)-1,3,3-tetramethyluronium hexafluorophosphate (known as HBTU) and 1-hyroxybenzotriazole hydrate (known as HOBT), dicyclohexylcarbodiimide (DCC), N-ethyl-N-(3-dimethylaminopropyl)- carbodiimide (EDC), diphenylphosphorylazide (DPPA), benzotriazol-1-yl-oxy-tris-(dimethylamino)phosphonium hexafluorophosphate (BOP) and the like, used in combination or singularly, may be utilized.

One skilled in the art understands that in the synthesis of compounds of the invention, one may need to protect various reactive functionalities on the starting compounds and intermediates while a desired reaction is carried out on other portions of the molecule. After the desired reactions are complete, or at any desired time, normally such protecting groups will be removed by, for example, hydrolytic or hydrogenolytic means. Such protection and deprotection steps are conventional in organic chemistry. One skilled in the art is referred to Protective Groups in Organic Chemistry, McOmie, ed., Plenum Press, NY, NY (1973); and, Protective Groups in Organic Synthesis, Greene, ed., John Wiley & Sons, NY, NY (1981) for the teaching of protective groups which may be useful in the preparation of compounds of the present invention.

By way of example only, useful amino-protecting groups may include, for example, C₁-C₁₀ alkanoyl groups such as formyl, acetyl, dichloroacetyl, propionyl, hexanoyl, 3,3-diethylhexanoyl, γ-chlorobutryl, and the like; C₁-C₁₀ alkoxycarbonyl and C₅-C₁₅ aryloxycarbonyl groups such as tert-butoxycarbonyl, benzyloxycarbonyl, allyloxycarbonyl, 4-nitrobenzyloxycarbonyl, fluorenylmethyloxycarbonyl and cinnamoyloxycarbonyl; halo-(C₁-C₁₀)-alkoxycarbonyl such as 2,2,2-trichloroethoxycarbonyl; and C₁-C₁₅ arylalkyl and alkenyl group such as benzyl, phenethyl, allyl, trityl, and the like. Other commonly used amino-protecting groups are those in the form of enamines prepared with β-keto-esters such as methyl or ethyl acetoacetate.

Useful carboxy-protecting groups may include, for example, C₁-C₁₀ alkyl groups such as methyl, tert-butyl, decyl; halo-C₁-C₁₀ alkyl such as 2,2,2-trichloroethyl, and 2-iodoethyl; C₅-C₁₅ arylalkyl such as benzyl, 4-methoxybenzyl, 4-nitrobenzyl, triphenylmethyl, diphenylmethyl; C₁-C₁₀ alkanoyloxymethyl such as acetoxymethyl, propionoxymethyl and the like; and groups such as phenacyl, 4-halophenacyl, allyl, dimethylallyl, tri-(C₁-C₃ alkyl) silyl, such as trimethylsilyl, β-p-toluenesulfonylethyl, β-p-nitrophenylthioethyl, 2,4,6-trimethylbenzyl, β-methylthioethyl, phthalimidomethyl, 2,4-dinitro-phenylsulphenyl, 2-nitrobenzhydryl and related groups.

Similarly, useful hydroxy protecting groups may include, for example, the formyl group, the chloroacetyl group, the benzyl group, the benzhydryl group, the trityl group, the 4-nitrobenzyl group, the trimethylsilyl group, the phenacyl group, the tert-butyl group, the methoxymethyl group, the tetrahydropyranyl group, and the like.

With respect to the preferred embodiment of an oligopeptide combined with vinblastine or desacetylvinblastine, the following Reaction Schemes illustrate the synthsis of the conjugates of the instant invention.

Reaction Scheme I illustrates preparation of conjugates of the oligopeptides of the instant invention and the vinca alkaloid cytotoxic agent vinblastine wherein the attachment of vinblastine is at the C-terminus of the oligopeptide. Furthermore, Scheme I illustrates a synthesis of conjugates wherein the C-4-position hydroxy moiety is reacetylated following the addition of the linker unit. Applicants have discovered that the desacetyl vinblastine conjugate is also efficacious and may be prepared by eliminating the steps shown in Reaction Scheme I of protecting the primary amine of the linker and reacting the intermediate with acetic anhydride, followed by deprotection of the amine. Conjugation of the oligopeptide at other positions and functional groups of vinblastine may be readily accomplished by one of ordinary skill in the art and is also expected to provide compounds useful in the treatment of prostate cancer.

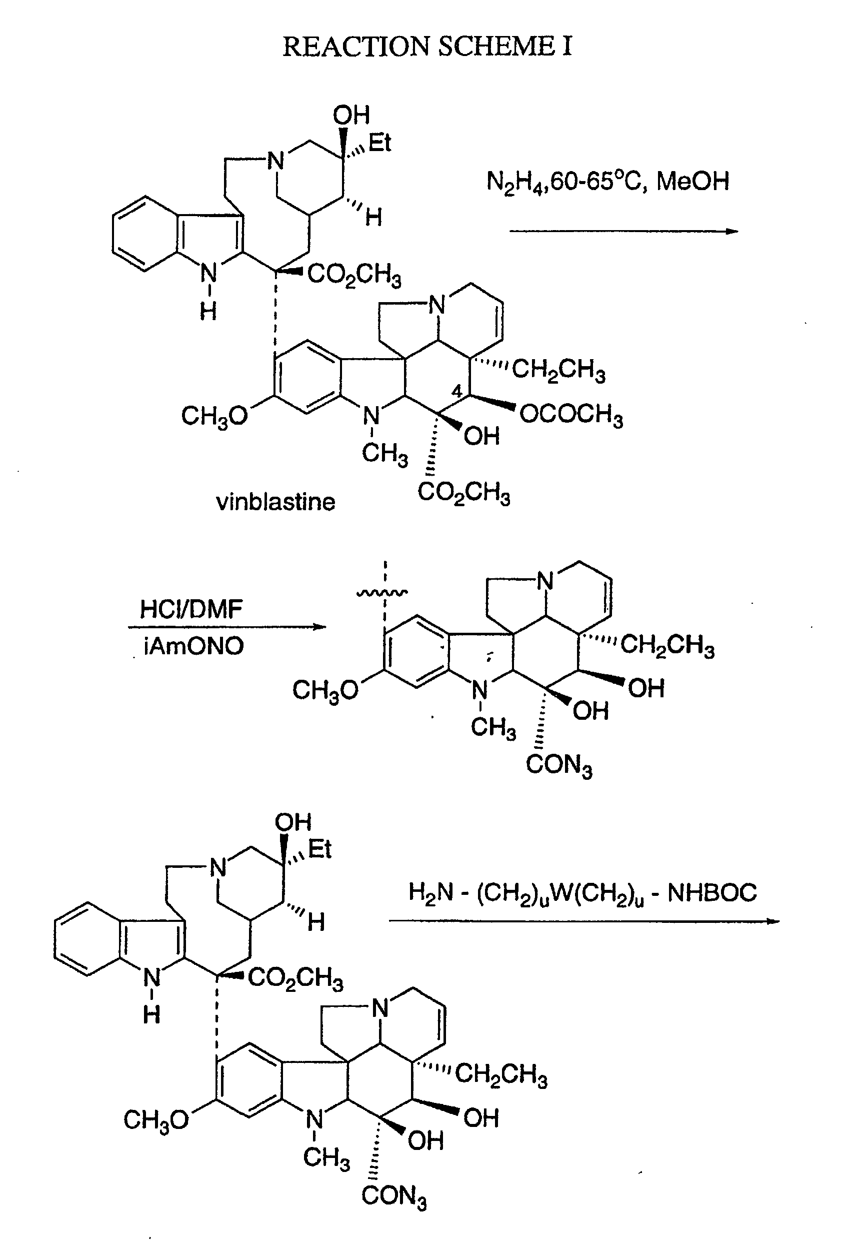

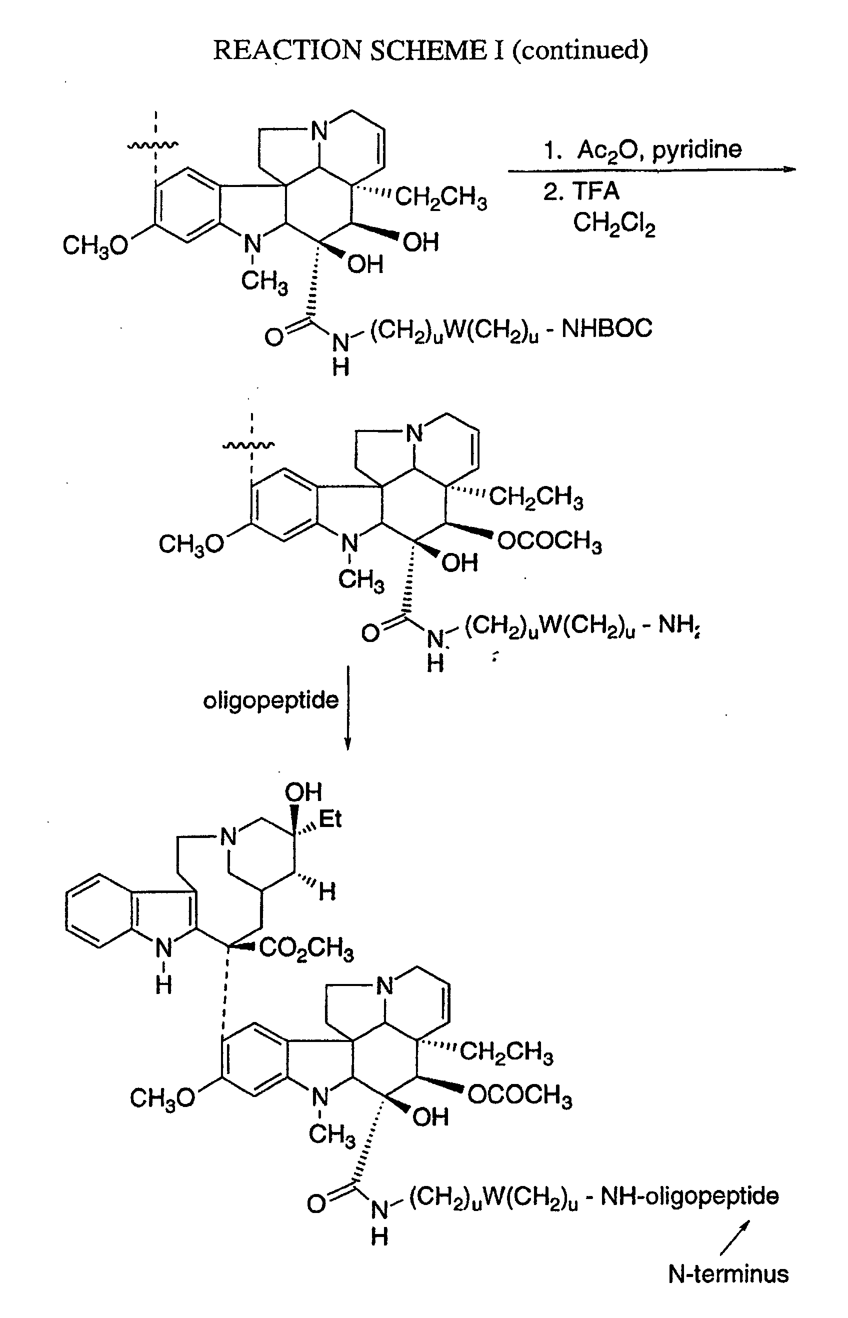

The oligopeptide-linker-cytotoxic agent conjugates of the invention are administered to the patient in the form of a pharmaceutical composition which comprises a conjugate of of the instant invention and a pharmaceutically acceptable carrier, excipient or diluent therefor. As used, "pharmaceutically acceptable" refers to those agents which are useful in the treatment or diagnosis of a warm-blooded animal including, for example, a human, equine, procine, bovine, murine, canine, feline, or other mammal, as well as an avian or other warm-blooded animal. The preferred mode of administration is parenterally, particularly by the intravenous, intramuscular, subcutaneous, intraperitoneal, or intralymphatic route. Such formulations can be prepared using carriers, diluents or excipients familiar to one skilled in the art. In this regard, See, e.g. Remington's Pharmaceutical Sciences, 16th ed., 1980, Mack Publishing Company, edited by Osol et al. Such compositions may include proteins, such as serum proteins, for example, human serum albumin, buffers or buffering substances such as phosphates, other salts, or electrolytes, and the like. Suitable diluents may include, for example, sterile water, isotonic saline, dilute aqueous dextrose, a polyhydric alcohol or mixtures of such alcohols, for example, glycerin, propylene glycol, polyethylene glycol and the like. The compositions may contain preservatives such as phenethyl alcohol, methyl and propyl parabens, thimerosal, and the like. If desired, the composition can include about 0.05 to about 0.20 percent by weight of an antioxidant such as sodium metabisulfite or sodium bisulfite.

As used herein, the term "composition" is intended to encompass a product comprising the specified ingredients in the specific amounts, as well as any product which results, directly or indirectly, from combination of the specific ingredients in the specified amounts.

For intravenous administration, the composition preferably will be prepared so that the amount administered to the patient will be from about 0.01 to about 1 g of the conjugate. Preferably, the amount administered will be in the range of about 0.2 g to about 1 g of the conjugate. The conjugates of the invention are effective over a wide dosage range depending on factors such as the disease state to be treated or the biological effect to be modified, the manner in which the conjugate is administered, the age, weight and condition of the patient as well as other factors to be determined by the treating physician. Thus, the amount administered to any given patient must be determined on an individual basis.

The following preparations and examples are provided to further illustrate the invention, and are not limiting.

### EXAMPLES

### EXAMPLE 1

Preparation of 4-*des*- Acetylvinblastine-23-(4'-aminomethylbicyclo-[2.2.2]octane) methylamide (BDAM-(dAc)vinblastine)

### Step A Preparation of 4-des- Acetylvinblastine-23-hydrazide

A sample of 3.99 g (4.38 mmol) of vinblastine sulfate (Sigma V-1377) was dissolved in 30.4 ml of 1:1 (v/v) absolute ethanol / anhydrous hydrazine, under N₂, and the solution was heated in an oil bath at 60-65°C for 23 hr. Upon cooling, the solution was evaporated to a thick paste, which was partitioned between 300ml of CH₂Cl₂ and 150 ml of saturated NaHCO₃. The aqueous layer was washed with 2 100-ml portions of CH₂Cl₂, and each of the 3 CH₂Cl₂ layers in turn was washed with 100 ml each of H2O (2X) and saturated NaCl (1X). The combined organic layers were dried over anhydrous Na₂SO4, and the solvent was removed *in vacuo* to yield, after drying 20 hr *in vacuo*, the title compound as a white crystalline solid. This material was dissolved in 82 ml of dry, degassed DMF for storage at -20°C until use (conc. 36 mg/ml).

### Step B Boc-4-aminomethylbicyclo-[2.2.2]octane carboxylic acid

A sample of 8.79 g (40.0 mmol) of 4-carboxybicyclo-[2.2.2]octanemethylamine hydrochloride salt suspended in 100 ml each of THF and H₂O was treated with 20.0 ml (14.6 g = 3.3 equiv.) of TEA, followed by 11.8 g (47.9 mmol) of BOC-ON reagent. All went into solution, and after stirring 24 hr the solution was concentrated *in vacuo* to a volume of about 50 ml and partitioned between 100 ml of ether and 300 ml of H₂O. After addition of about 2 ml of TEA the aqueous layer was washed with ether (3X), each ether in turn washed with H₂O, and the combined aqueous layer was acidified with 5% KHSO₄ to give the title compound as a white solid, isolated by filtration and drying *in vacuo*.

### Step C Boc-4-aminomethylbicyclo-[2.2.2]octane carboxamide

A stirred solution under N₂ of 12.0 g (42.5 mmol) of the product from step B in 100 ml of DMF was treated with 8.0 g (49.3 mmol) of carbonyldiimidazole. After 30 min the DMF was evaporated *in vacuo* to afford 50-60 ml of a light brown paste, which was stirred and treated with 70 ml of cone. NH₄OH rapidly added. The initial solution turned to a white paste within 30 min, after which H₂O was added up to a total volume of 400 ml to complete precipitation of product, which was triturated and isolated by filtration and washing with H₂O, and dried *in vacuo* to yield the title compound as a white solid.

### Step D Boc-4-aminomethylbicyclo-[2.2.2]octane nitrile

A solution of 7.52 g (26.6 mmol) of the product from step C in 50 ml of CH₂Cl₂ and 80 ml of anhydrous pyridine was treated with 11.12 g of (methoxycarbonylsulfamoyl)-triethylammonium hydroxide inner salt (Burgess reagent) in 1-g portions over 5 min. After stirring for 1.5 hr, TLC (90-10-1, CHCl₃-CH₃OH-H₂O) showed complete conversion to product, and the solution was evaporated to give a paste, to which H2O was added, up to 400 ml, with trituration and stirring to afford, after standing 20 hr at 0°C, filtration and drying *in vacuo*, the title compound as a white solid.

### Step E Boc-4-aminomethylbicyclo-[2.2.2]octane methylamine

A solution of 6.75 g (25.5 mmol) of the product from step D in 200 ml of CH₃OH plus 4 ml of HOAc and 2 ml of H₂O was hydrogenated over 1.63 g of PtO₂ in a Parr shaker at 55 psi for 22 hr. The catalyst was removed by filtration through Celite, and the filtrate was concentrated *in vacuo* to an oily residue, which was flushed/evaporated with CH₃OH (1X) and CH₂Cl₂ (2X). Product began to crystallize toward the end of the evaporation, and ether (up to 300 ml) was added to complete the precipitation. The white solid was triturated and isolated by filtration and washing with ether to give, after drying *in vacuo*, the title compound as the acetate salt.
400 Mhz 1H-NMR (CDCl₃): δ (ppm, TMS) 4.5 (1s, Boc-NH); 2.9 (2br d, -CH2-NH-Boc); 2.45 (2br s, -CH2-NH2); 2.03 (3s, CH3COOH);1.45 (9s, Boc); 1.40 (12s, ring CH2).

### Step E Preparation of 4-des- Acetylvinblastine-23-(4'-aminomethylbicyclo-[2.2.2]octane) methylamide (BDAM-(dAc)vinblastine)

A 30-ml aliquot of the above DMF solution of 4-*des*-acetylvinblastine-23-hydrazide (1.41 mmol), cooled to -15°C under Argon, was converted to the azide *in situ* by acidification with 4M HCl in dioxane to pH < 1.5 (moistened 0-2.5 range paper), followed by addition of 0.27 ml (1.3 equiv) of isoamyl nitrite and stirring for 1 hr at 10-15°C. The pH was brought to 7 by the addition of DIEA, and a slurry of 1.27 g (3.8 mmol) of the Boc diamine product from step D above in 20 ml of DMF was then added, and the reaction was allowed to warm slowly to 15-20°C over 2 hr, at which point coupling was complete, as monitored by analytical HPLC (A = 0.1% TFA/H₂O; B = 0.1% TFA/CH₃CN). The solvent was removed *in vacuo* and the residue partitioned between EtOAc and 5% NaHCO₃, the organic layer washed with 5% NaCl, and the aqueous layers back-extracted with CH₂Cl₂ to assure removal of the intermediary Boc-BDAM-(dAc)vinblastine. The combined organic layers were dried over Na₂SO₄, the solvent was removed under reduced pressure, and the residue, after flush/evaporation twice from CH₂Cl₂, was dissolved in 30 ml of CH₂Cl₂ and treated with 30 ml of TFA for 30 min. The solvents were rapidly removed *in vacuo,* and the residue was dissolved in 300 ml of 10% HOAc for purification by preparative HPLC in 5 portions on a Waters C4 Delta-Pak column 15µM 300A (A = 0.1 % TFA/H₂O; B = 0.1 % TFA/CH₃CN), gradient elution 95 --> 70% A / 60 min, isocratic 70% / 20 min. Homogeneous fractions (evaluated by HPLC, system A, 95 --> 50% A) from the five runs were pooled and concentrated *in vacuo*, followed by freeze-drying to give of the title compound as the lyophilized TFA salt.

| HPLC conditions, system A: | |
|---|---|
| Column... | Vydac 15 cm #218TP5415, C18 |
| Eluant... | Gradient (A --> B) over 45 min. A = 0.1% TFA/H₂O, B = 0.1% |

| TFA/acetonitrile | |
|---|---|
| Flow... | 1.5 ml/min. |

Retention time: BDAM (dAc) vinblastine 23.5 min. (95% --> 50% A)
97% purity
High Resolution ES/FT-MS: 905.63

| Compound content by elemental analysis = 0.714 µmol/mg: | |
|---|---|
| N (calc) = 9.28 | N (found) = 6.00 |

### EXAMPLE 2

### Preparation of 4-des- Acetylvinblastine-23-(N-Acetyl-Ser-Ser-Ser-Chg-Gln-Ser-Val-BDAM) amide acetate salt (SEQ.ID.NO.: 36)

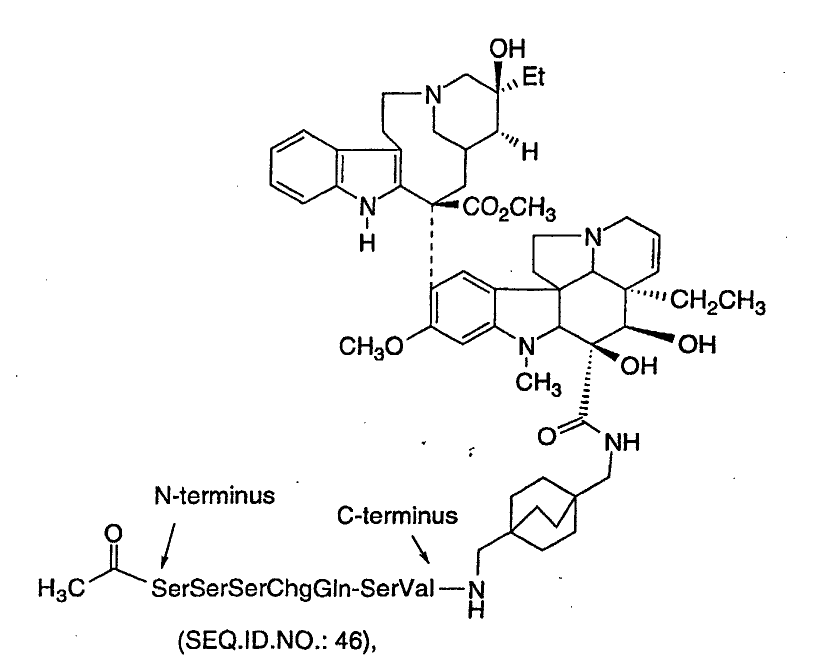

### Step A: N-Acetyl-Ser-Ser-Ser-Chg-Gln-Ser-Val-PAM Resin (SEQ.ID.NO.: 46)

Starting with 0.5 mmole (0.68 g) of Boc-Val-PAM resin, the protected peptide was synthesized on a ABI model 430A peptide synthesizer. The protocol used a 4-fold excess (2.0 mmol) of each of the following protected amino acids: Boc-Ser(Bzl)-OH, Boc-Gln-OH, Boc-Chg-OH; and acetic acid (2 couplings). During each coupling cycle Boc protection was removed using TFA, followed by neutralization with DIEA. Coupling was achieved using DCC and HOBt activation in N-methyl-2-pyrrolidinone. At the completion of the synthesis, the peptide resin was dried to yield the title compound.

### Step B: N-Acetyl-Ser-Ser-Ser-Chg-Gln-Ser-Val-OH (SEQ.ID.NO.: 46)

Three 0.5-mmol runs of the above peptide-resin (3.5 g) were combined and treated with liquid HF (65 ml) for 1.5 hr at 0°C in the presence of anisole (6 ml). After evaporation of the HF, the residue was washed with ether, filtered and leached with 150 ml of DMF in several portions, adding DIEA to pH ∼8, followed by removal of the DMF *in vacuo* to a volume of 100 ml. The concentration was determined as *ca*. 11.7 mg/ml (by weighing the dried resin before and after leaching. The sample purity was determined as 96% by HPLC. The solution was used directly for conjugation with BDAM-(dAc)vinblastine.

### Step C: 4-Des- acetylvinblastine-23-(N-Acetyl-Ser-Ser-Ser-Chg-Gln-Ser-Val-BDAM) amide acetate salt

To 58 ml (equivalent to 0.875, mmol of peptide) of the solution from step B was added 530 mg (0.520 mmol) of BDAM-(dAc)vinblastine, prepared as above, under N2, cooling to 0°C, and the pH was adjusted to ∼8 (moistened 5-10 range pH paper) with DIEA. Then 0.134 ml (0.62 mmol) of DPPA was added, followed by stirring at 0-5°C until completion of the coupling as monitored by analytical HPLC (A = 0.1% TFA/H₂O; B = 0.1% TFA/CH₃CN), maintaining the pH at ≥ 7 by periodic addition of DIEA. After 24 hr the reaction was worked up by addition of 10 ml of H₂O, stirring 1 hr and concentration to small volume *in vacuo,* then dissolution in *ca.* 100 ml of 10% HOAc/5% CH₃CN, adjustment of the pH to 5 with NH₄HCO₃, filtration to remove insolubles, and preparative HPLC in 3 portions on a Waters C4 Delta-Pale column 15µM 300A (A = 0.1% NH₄HCO₃/H₂O; B = CH₃CN), gradient elution 95 --> 40% A / 70 min. Fractions from each run containing product were pooled, acidified to pH 3 with glacial HOAc, concentrated *in vacuo* to a volume of ∼50 ml, and purified by preparative HPLC on a Waters C18 Delta-Pak column 15µM 300A (A = 0.1% TFA/H₂O; B = 0.1% TFA/CH₃CN), gradient elution 95 --> 70% A / 60 min, isocratic 70% / 20 min. Homogeneous fractions (evaluated by HPLC, system A, 95 --> 50% A) from all three runs were pooled and concentrated to a volume of ∼100 ml., diluted with 5% CH₃CN, and passed through AG4X4 ion exchange resin (acetate cycle), followed by freeze-drying to give the title compound as a lyophilized powder.

| HPLC conditions, system A: | |
|---|---|
| Column... | Vydac 15 cm #218TP5415, C18 |
| Eluant... | Gradient (A --> B) over 45 min. A = 0.1% TFA/H2O, B = 0.1% |

| TFA/acetonitrile | |
|---|---|
| Flow... | 1.5 ml/min. |

Retention times: BDAM (dAc) vinblastine 23.5 min.
N-Acetyl-Ser-Ser-Ser-Chg-Gln-Ser-Val-OH 14.5 min.
4-*Des*- acetylvinblastine-23-( N-Acetyl-Ser-Ser-Ser-Chg-Gln-Ser-Val-BDAM) amide 29.5 min.
High Resolution ES/FT-MS: 1662.03

| Amino Acid Compositional Analysis¹ (theory/found): | | | |
|---|---|---|---|
| ²Ser4/3.6 | ³Glu 1/2.10 | ⁴Val 1/0.7 | Chg 1/0.95 |
| Peptide content 0.504 µmol/mg | | | |

| | | | |
|---|---|---|---|
| Note: ¹20 hr, 100°C, 6N HCl | | | |
| ²Uncorrected | | | |
| ³Gln converted to Glu | | | |
| ⁴Incomplete hydrolysis | | | |

### EXAMPLE 3

### Preparation of 4-des- Acetylvinblastine-23-(N-methoxy-diethylene-oxyacetyl-4-trans-L-Hyp-Ser-Ser-Chg-Gln-Ser-Val-BDAM) amide_ acetate salt (SEQ.ID.NO.: 70)

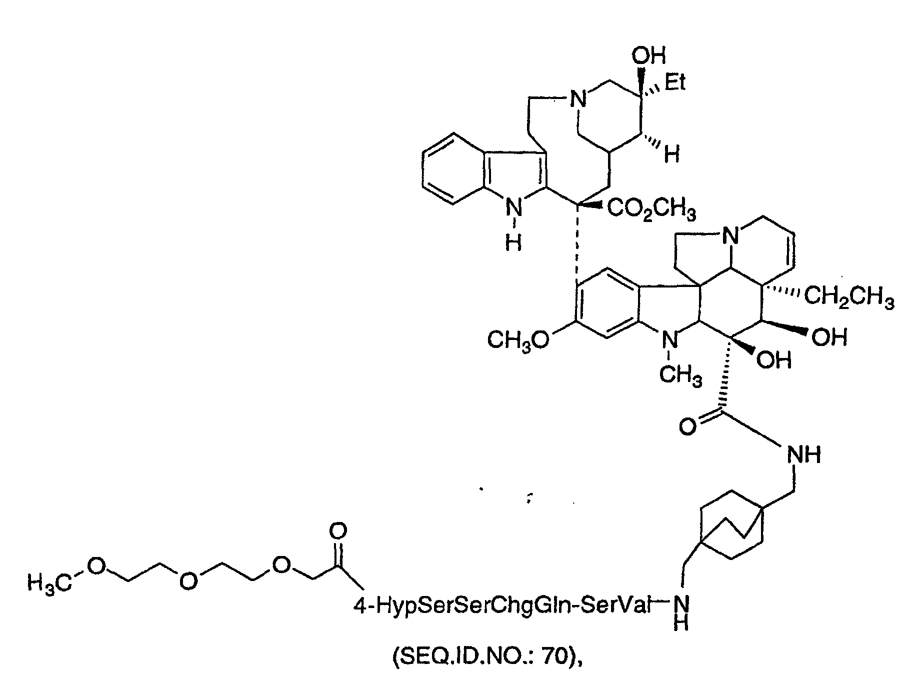

### Step A: N-methoxydiethyleneoxyacetyl-4-trans-L-Hyp-Ser-Ser-Chg-Gln-Ser-Val-PAM Resin (SEQ.ID.NO.: 70)

Starting with 0.5 mmole (0.68 g) of Boc-Val-PAM resin, the protected peptide was synthesized on a ABI model 430A peptide synthesizer. The protocol used a 4-fold excess (2.0 mmol) of each of the following protected amino acids: Boc-Ser(Bzl)-OH, Boc-Gln-OH, Boc-Chg-OH, Boc-4-trans-Hyp(Bzl)-OH; and 2-[2-(2-methoxyethoxy)-ethoxy]acetic acid (2 couplings). During each coupling cycle Boc protection was removed using TFA, followed by neutralization with DIEA. Coupling was achieved using DCC and HOBt activation in N-methyl-2-pyrrolidinone. At the completion of the synthesis, the peptide resin was dried to yield the title compound.

### Step B: N-methoxydiethyleneoxyacetyl-4-trans-L-Hyp-Ser-Ser-Chg-Gln-Ser-Val-OH (SEQ.ID.NO.: 70)

Two 0.5-mmol runs of the above peptide-resin (2.4 g) were combined and treated with liquid HF (40 ml) for 1.5 hr at 0°C in the presence of anisole (4 ml). After evaporation of the HF, the residue was washed with ether, filtered and leached with 150 ml of H₂O in several portions, followed by preparative HPLC on a Waters C18 Delta-Pak column 15µM 100A (A = 0.1% TFA/H₂O; B = 0.1% TFA/CH₃CN), gradient elution 95 --> 70% A / 70 min, and pooling of homogeneous fractions and freeze drying to give the title compound as lyophilized powder. The sample purity was determined as 99% by HPLC.

### Step C: 4-des- Acetylvinblastine-23-(N-methoxydiethyleneoxyacetyl-4-trans-L-Hyp-Ser-Ser-Chg-Gln-Ser-Val-BDAM) amide acetate salt

Samples of 440 mg (0.47 mmol) of the peptide from step B and 340 mg (0.33 mmol) of BDAM-(dAc)vinblastine, prepared as above, were dissolved in 25 ml of DMF under N₂, cooling to 0°C. Then 85 mg (0.63 mmol) of 1-hydroxy-7-azabenzotriazole (HOAt) was added, and the pH was adjusted to 6.5-7 (moistened 5-10 range pH paper) with 2,4,6-collidine, followed by addition of 117 mg (0.61 mmol) of 1-(3-dimethylaminopropyl)-3-ethylcarbodiimide hydrochloride (EDC). Stirring was continued at 0-5°C until completion of the coupling as monitored by analytical HPLC (A = 0.1 % TFA/H₂O; B = 0.1 % TFA/CH₃CN), maintaining the pH at 6.5-7 by periodic addition of 2,4,6-collidine. After 3 hr the reaction was worked up by addition of ∼10 ml of H₂O, stirring 1 hr and concentration to small volume *in vacuo*, then dissolution in ca. 70 ml of 5% HOAc. and preparative HPLC on a Waters C18 Delta-Pak column 15µM 300A (A = 0.1 % TFA/H₂O; B = 0.1 % TFA/CH₃CN), gradient elution 95 --> 40% A / 70 min). Homogeneous fractions (evaluated by HPLC, system A, 95 --> 50% A) from all three runs were pooled and concentrated to a volume of ∼50 ml and passed through AG4X4 ion exchange resin (acetate cycle), followed by freeze-drying to give the title compound as a lyophilized powder.

| HPLC conditions, system A: | |
|---|---|
| Column... | Vydac 15 cm #218TP5415, C18 |
| Eluant... | Gradient (A --> B) over 45 min. A = 0.1% TFA/H₂O, B = 0.1 % |

| TFA/acetonitrile | |
|---|---|
| Flow... | 1.5 ml/min. |

Retention times: BDAM (dAc) vinblastine 23.5 min.
N-methoxydiethyleneoxyacetyl-4-trans-L-Hyp-Ser-Ser-Chg-Gln-Ser-Val-OH 16.2 min.
4-*des*- Acetylvinblastine-23-(N-methoxydiethyleneoxyacetyl-4-trans-L-Hyp-Ser-Ser-Chg-Gln-Ser-Val-BDAM) amide 29.6 min.
High Resolution ES/FT-MS: 1805.95

| Amino Acid Compositional Analysis¹ (theory/found): | | | | |
|---|---|---|---|---|
| ²Ser3/1.7 | ³Glu 1/1.01 | ⁴Val 1/0.93 | Chg 1/0.98 | Hyp 1/1.01 |
| Peptide content = 0.497 µmol/mg | | | | |

| | | | | |
|---|---|---|---|---|
| Note: ¹20 hr, 100°C, 6N HCl | | | | |
| ²Uncorrected | | | | |
| ³Gln converted to Glu | | | | |
| ⁴Incomplete hydrolysis | | | | |

Table 3 shows other peptide-vinca drug conjugates that were prepared by the procedures described in Examples 2 and 3, but utilizing the appropriate amino acid residues and blocking group acylation. Unless otherwise indicated, the acetate salt of the conjugate was prepared and tested.

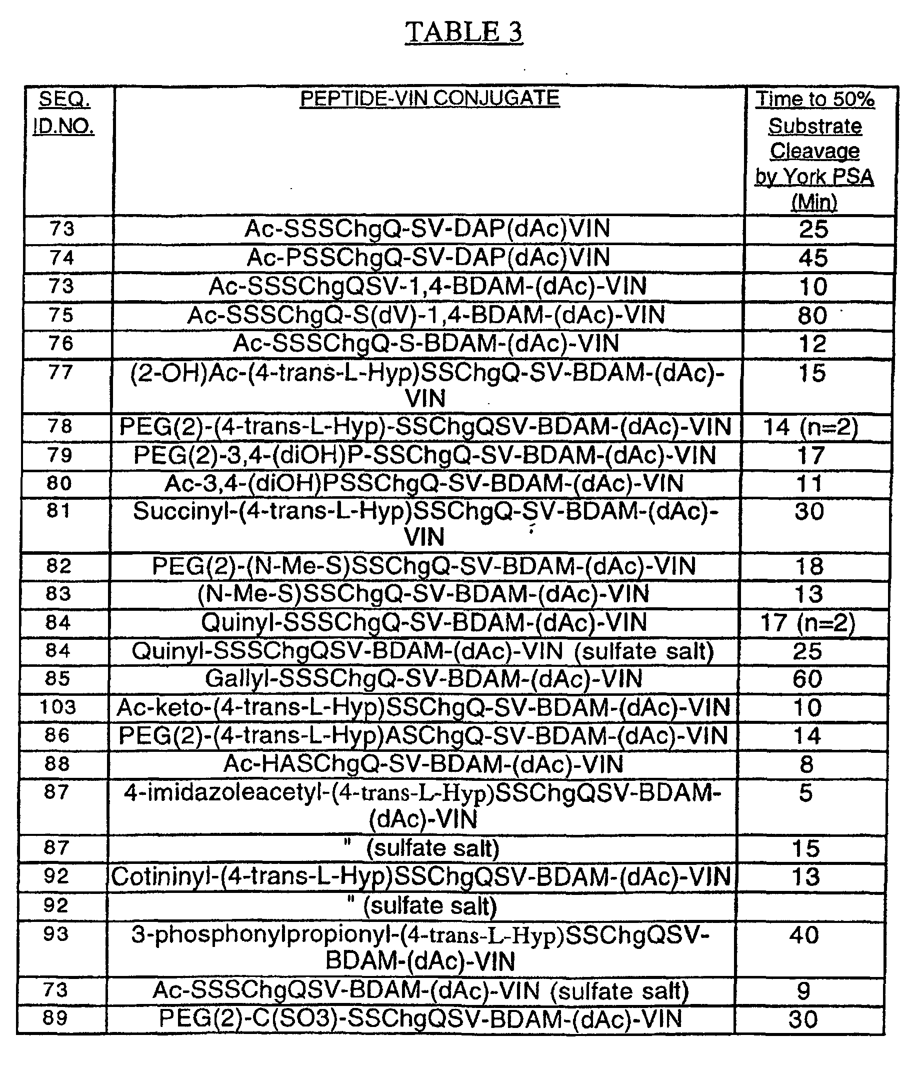

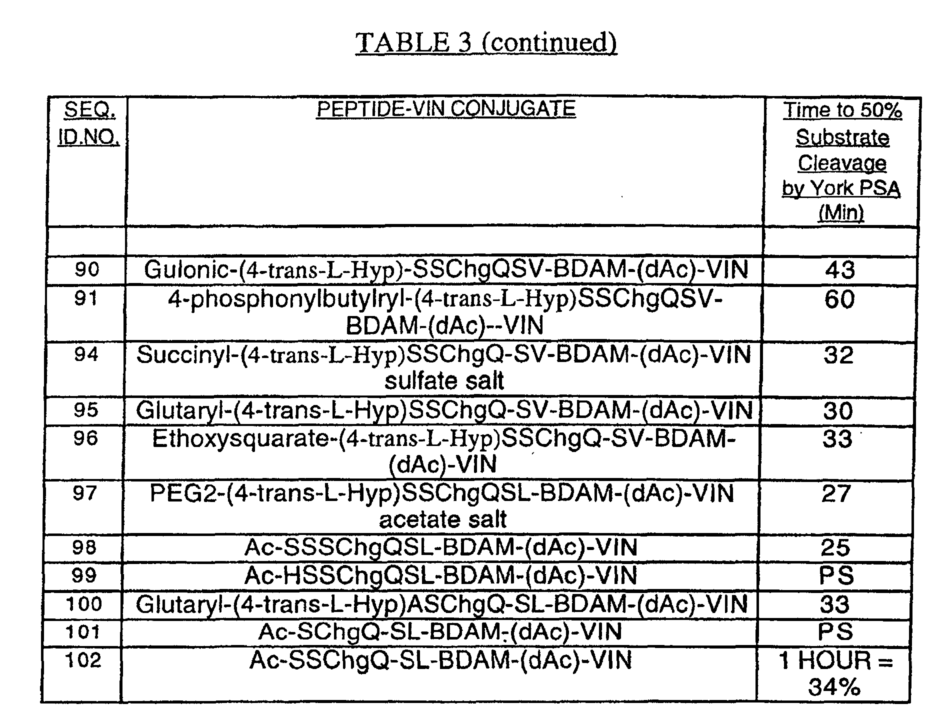

4-trans-L-Hyp is *trans*-4-hydroxy-L-proline
C(SO3) is -NHCH(CH₂CH₂SO₃⁻)CO₂-3,4-(diOH)P is 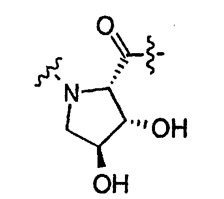 Ac-keto-(4-trans-L-Hyp) is 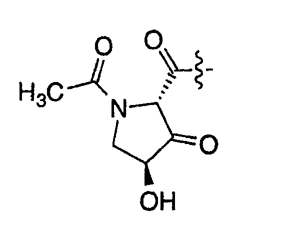 PS = partially soluble
when n > 1; value is an average

### EXAMPLE 4

### Assessment of the Recognition of Oligopeptide-Vinca Drug Conjugates by Free PSA :

The conjugates prepared as described in Example 3 were individually dissolved in PSA digestion buffer (50 mM tris(hydroxymethyl)-aminomethane pH7.4, 140 mM NaCl) and the solution added to PSA at a molar ration of 100 to 1. Alternatively, the PSA digestion buffer utilized is 50 mM tris(hydroxymethyl)-aminomethane pH7.4, 140 mM NaCl. The reaction is quenched after various reaction times by the addition of trifluoroacetic acid (TFA) to a final 1% (volume/volume). Alternatively the reaction is quenched with 10mM ZnCl₂. The quenched reaction was analyzed by HPLC on a reversed-phase C18 column using an aqueous 0.1%TFA/acctonitrile gradient. The results of the assessment are shown in Table 3. Table 3 shows the amount of time (in minutes) required for 50% cleavage of the noted oligopeptide-cytotoxic agent conjugates with enzymatically active free PSA. Unless otherwise indicated, the acetate salt of the conjugate was tested.

### EXAMPLE 5

### In vitro Assay of Cytotoxicity of Peptidyl Derivatives of Vinca Drugs

The cytotoxicities of the cleaveable oligopeptide-vinca drug conjugates, prepared as described in Example 3, against a line of cells which is known to be killed by unmodified vinca drug was assessed with an Alamar Blue assay. Specifically, cell cultures of LNCap prostate tumor cells, Colo320DM cells (designated C320) or T47D cells in 96 well plates was diluted with medium containing various concentrations of a given conjugate (final plate well volume of 200µl). The cells were incubated for 3 days at 37°C, 20µl of Alamar Blue is added to the assay well. The cells were further incubated and the assay plates were read on a EL-310 ELISA reader at the dual wavelengths of 570 and 600 nm at 4 and 7 hours after addition of Alamar Blue. Relative percentage viability at the various concentration of conjugate tested was then calculated versus control (no conjugate) cultures. Results of this assay are shown in Table 4. Unless otherwise indicated, the acetate salt of the conjugate was tested.

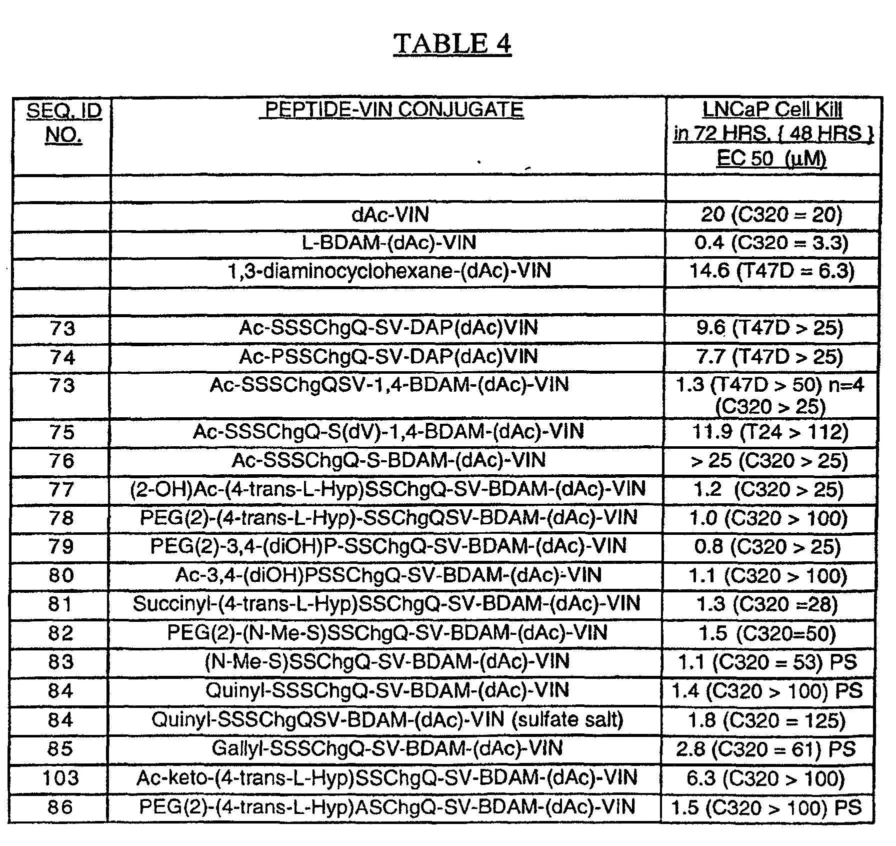

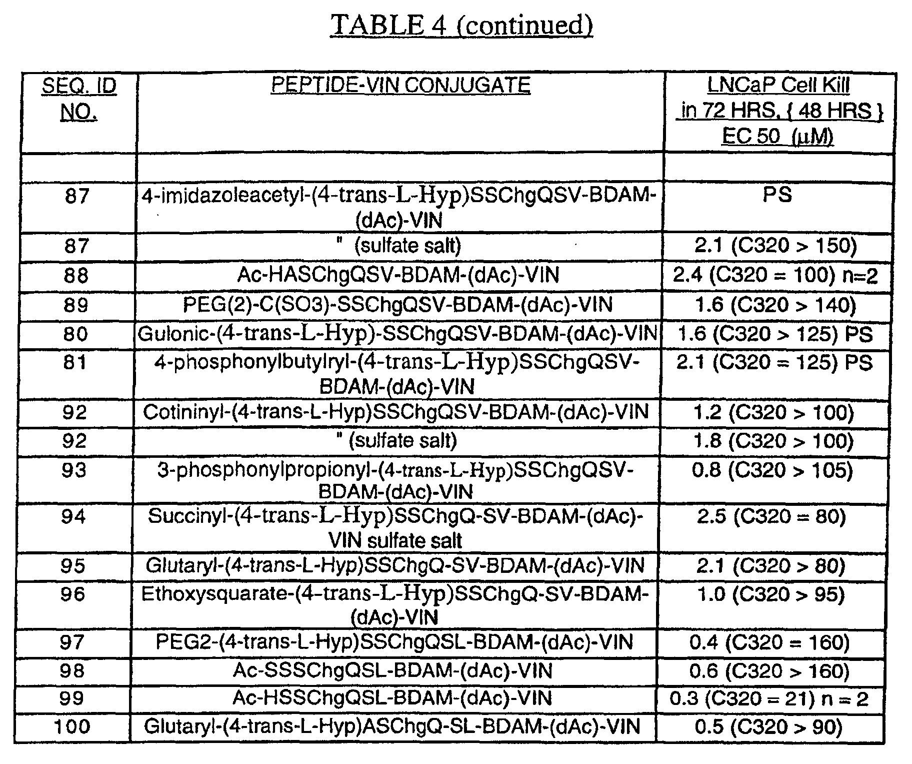

### EXAMPLE 6

### In vivo Efficacy of Peptidyl -Cytotoxic Agent Conjugates

LNCaP.FGC or C320 cells are trypsinized, resuspended in the growth medium and centifuged for 6 mins. at 200xg. The cells are resuspended in serum-free α―MEM and counted. The appropriate volume of this solution containing the desired number of cells is then transferred to a conical centrifuge tube, centrifuged as before and resuspended in the appropriate volume of a cold 1:1 mixture of α―MEM-Matrigel. The suspension is kept on ice until the animals are inoculated.

Harlan Sprague Dawley male nude mice (10-12 weeks old) are restrained without anesthesia and are inoculated with 0.5 mL of cell suspension on the left flank by subcutaneous injection using a 22G needle. Mice are either given approximately 5x10⁵ DuPRO cells or 1.5x10⁷ LNCaP.FGC cells.

Following inoculation with the tumor cells the mice are treated under one of two protocols:

### Protocol A:

One day after cell inoculation the animals are dosed with a 0.1-0.5 mL volume of test conjugate, vinca drug or vehicle control (sterile water). Dosages of the conjugate and vinca drug are initially the maximum non-lethal amount, but may be subsequently titrated lower. Identical doses are administered at 24 hour intervals for 5 days. After 10 days, blood samples are removed from the mice and the serum level of PSA is determined. Similar serum PSA levels are determined at 5-10 day intervals. At the end of 5.5 weeks the mice are sacrificed and weights of any tumors present are measured and serum PSA again determined.The animals' weights are determined at the beginning and end of the assay.

### Protocol B:

Ten days after cell inoculation,blood samples are removed from the animals and serum levels of PSA are determined. Animals are then grouped according to their PSA serum levels. At 14-15 days after cell inoculation, the animals are dosed with a 0.1-0.5 mL volume of test conjugate, vinca drug or vehicle control (sterile water). Dosages of the conjugate and vinca drug are initially the maximum non-lethal amount, but may be subsequently titrated lower. Identical doses are administered at 24 hour intervals for 5 days. Serum PSA levels are determined at 5-10 day intervals. At the end of 5.5 weeks the mice are sacrificed, weights of any tumors present are measured and serum PSA again determined. The animals' weights are determined at the beginning and end of the assay.

### EXAMPLE 7

*In vitro* determination of proteolytic cleavage of conjugates by endogenous non-PSA proteases

### Step A: Preparation of proteolytic tissue extracts

All procedures are carried out at 4°C. Appropriate animals are sacrificed and the relevant tissues are isolated and stored in liquid nitrogen. The frozen tissue is pulverized using a mortar and pestle and the pulverized tissue is transfered to a Potter-Elvejeh homogenizer and 2 volumes of Buffer A (50 mM Tris containing 1.15% KCl, pH 7.5) are added. The tissue is then disrupted with 20 strokes using first a lose fitting and then a tight fitting pestle. The homogenate is centrifuged at 10,000 x g in a swinging bucket rotor (HB4-5), the pellet is discarded and the re-supernatant centrifuged at 100,000 x g (Ti 70). The supernatant (cytosol) is saved.

The pellet is resuspended in Buffer B (10 mM EDTA containing 1.15% KCl, pH 7.5) using the same volume used in step as used above with Buffer A. The suspension is homogenized in a dounce homogenizer and the solution centrifuged at 100,000x g. The supernatant is discarded and the pellet resuspended in Buffer C (10 mM potassium phosphate buffer containing0.25 M sucrose, pH 7.4), using 1/2 the volume used above, and homogenized with a dounce homogenizer.

Protein content of the two solutions (cytosol and membrane) is determine using the Bradford assay. Assay aliquots are then removed and frozen in liquid N₂. The aliquots are stored at -70°C.

### Step B: Proteolytic cleavage assay

For each time point, 20 microgram of peptide-vinca drug conjugate and 150 micrograms of tissue protein, prepared as described in Step A and as determined by Bradford in reaction buffer are placed in solution of final volume of 200 microliters in buffer (50 mM TRIS, 140 mM NaCl, pH 7.2). Assay reactions are run for 0, 30, 60, 120, and 180 minutes and are then quenched with 9 microliters of 0.1 M ZnCl₂ and immediately placed in boiling water for 90 seconds. Reaction products are analyzed by HPLC using a VYDAC C18 15 cm column in water / acetonitrile (5% to 50% acetonitrile over 30 minutes).

## Claims

1. A conjugate which is useful for the treatment of prostate cancer which comprises a vinca alkaloid cytotoxic agent attached to an oligopeptide, wherein the oligopeptide comprises a sequence of amino acids that is selectively proteolytically cleaved by free prostate specific antigen and wherein the means of attachment is through a diamine chemical linker which comprises a cycloalkyl or bicycloalkyl moiety,
or the pharmaceutically acceptable salt thereof, whereby the oligopeptide does not include semenogelin I, semenogelin II, fibronectin and IGFBP-3.

2. The conjugate according to Claim 1 wherein the cytotoxic agent is selected from the following cytotoxic agents:
a) vinblastine,
b) 4-desacetylvinblastine,
c) vincristine,
d) leurosidine, and
e) vindesine,
or the pharmaceutically acceptable salt thereof.

3. The conjugate according to Claim 2 wherein the cytotoxic agent is selected from vinblastine and 4-desacetylvinblastine.

4. The conjugate according to Claim 1 wherein the oligopeptide comprises an oligomer selected from:
a)
b)
c)
d)
e)
f)
g)
h)
i)
j)
k)
l)
m)
n)
o)
p)
q)
r)
s)
t)
u)
v)
w)
X)
y)
z)
wherein Haa is a cyclic amino acid substituted with a hydrophilic moiety, hArg is homoarginine, Xaa is any amino acid, Cha is cyclohexylalanine and Chg is cyclohexylglycine.

5. The conjugate according to Claim 1 wherein the oligopeptide comprises an oligomer selected from: and wherein 4-Hyp is 4-hydroxyproline, 3,4-DiHyp is 3,4-dihydroxyproline, N-methyl-Ser is cyclohexylalanine and Chg is cyclohexylglycine.

6. The conjugate according to Claim 1 of the formula I: 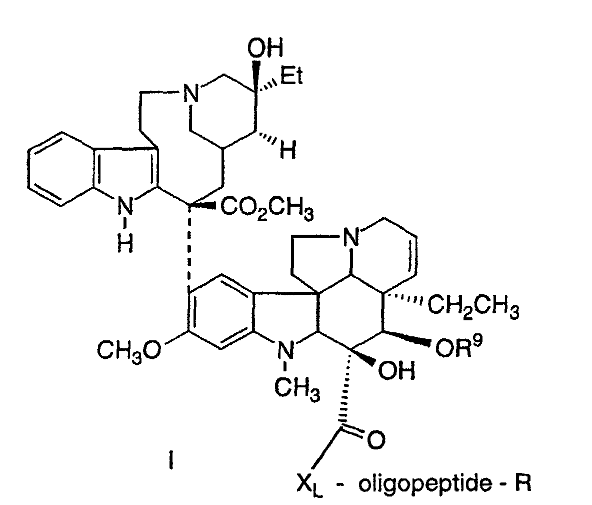 wherein:
oligopeptide is an oligopeptide which is specifically recognized by the free prostate specific antigen (PSA) and is capable of being proteolytically cleaved by the enzymatic activity of the free prostate specific antigen,
X_{L} is -NH-(CH₂)ᵤ-W-(CH₂)ᵤ-NH-
R is selected from
a) hydrogen,
b) -(C=O)R^{1a},
c) 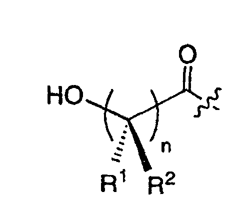
d) 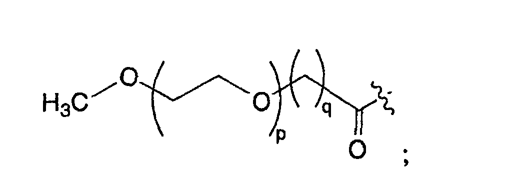
e) 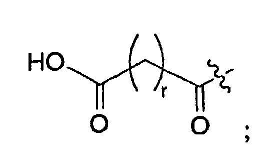
f) ethoxysquarate; and
g) cotininyl;
R¹ and R² are independently selected from: hydrogen, OH, C₁-C₆ alkyl, C₁-C₆ alkoxy, C₁-C₆ aralkyl and aryl;
R^{1a} is C₁-C₆-alkyl or aryl,
R¹⁹ is hydrogen, (C₁-C₃ alkyl)-CO, or chlorosubstituted (C₁-C₃ alkyl)-CO;
W is selected from cyclopentyl, cyclohexyl, cycloheptyl or bicyclo[2.2.2]octanyl;
n is 1, 2, 3 or 4;
p is zero or an integer between 1 and 100;
q is 0 or 1, provided that if p is zero, q is 1;
r is 1, 2 or 3;
t is 3 or 4;
u is 0, 1, 2 or 3,
or a pharmaceutically acceptable salt thereof.

7. The conjugate according to Claim 6 wherein:
oligopeptide is an oligomer that comprises an amino acid sequence selected from:
a)
b)
c)
d)
e)
f)
g)
h)
i)
j)
k)
l)
m)
n)
o)
p)
q)
r)
s)
t)
u)
v)
w)
x)
) y)
z)
wherein Haa is a cyclic amino acid substituted with a hydrophilic moiety, hArg is homoarginine, Xaa is any amino acid, Cha is cyclohexylalanine and Chg is cyclohexylglycine;
or an optical isomer or pharmaceutically acceptable salt thereof.

8. The conjugate according to Claim 7 wherein:
Xaa is alanine, serine or isoleucine; and
Haa is trans-4-hydroxy-L-proline;
or an optical isomer or pharmaceutically acceptable salt thereof.

9. The conjugate according to Claim 8 which is selected from: 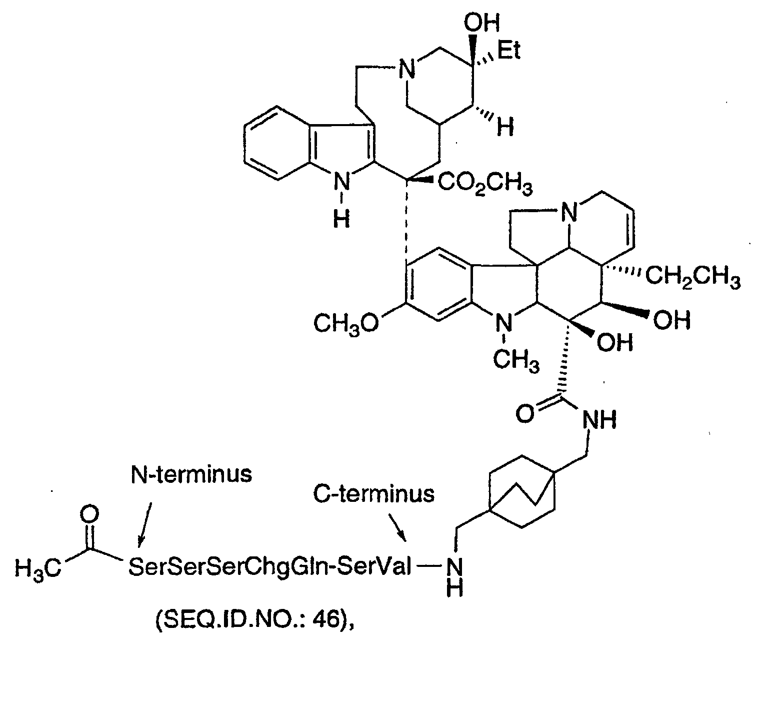 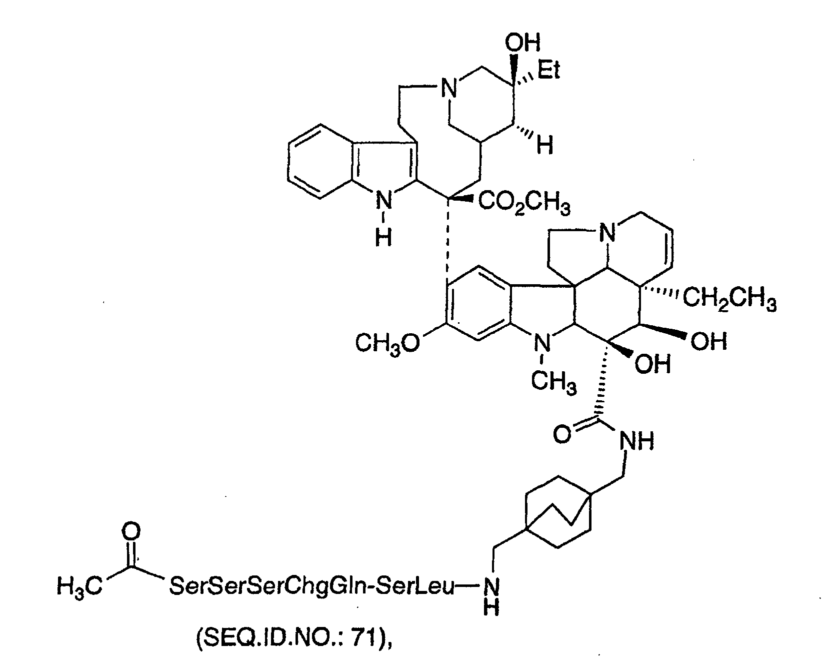 or a pharmaceutically acceptable salt or optical isomer thereof.

10. The conjugate according to Claim 8 which is selected from: 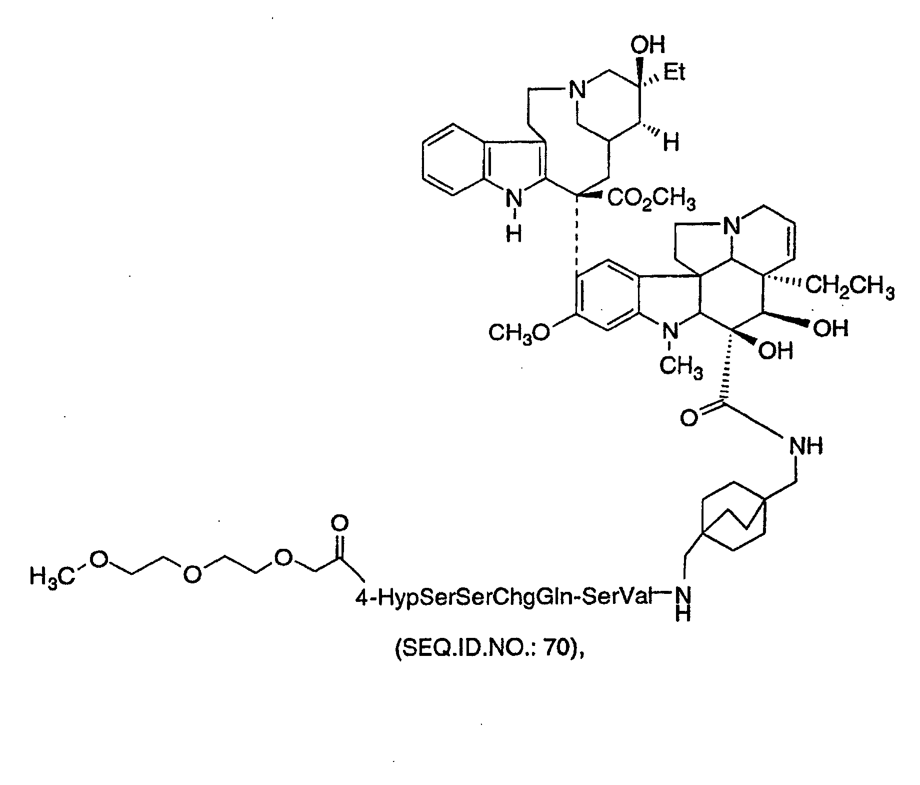 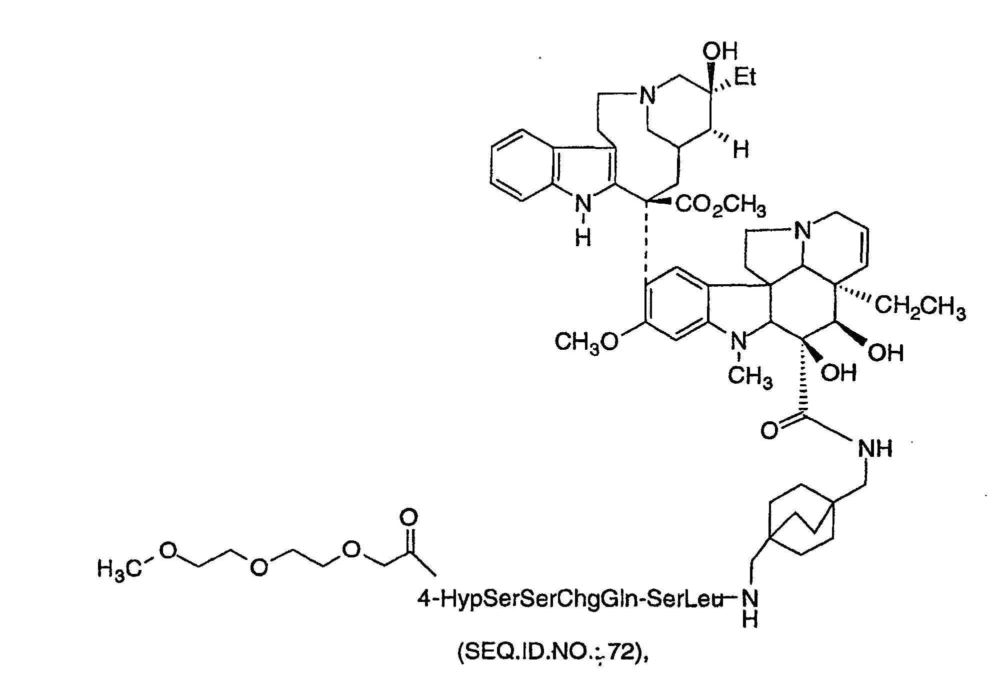 or a pharmaceutically acceptable salt or optical isomer thereof.

11. A compound which is selected from: 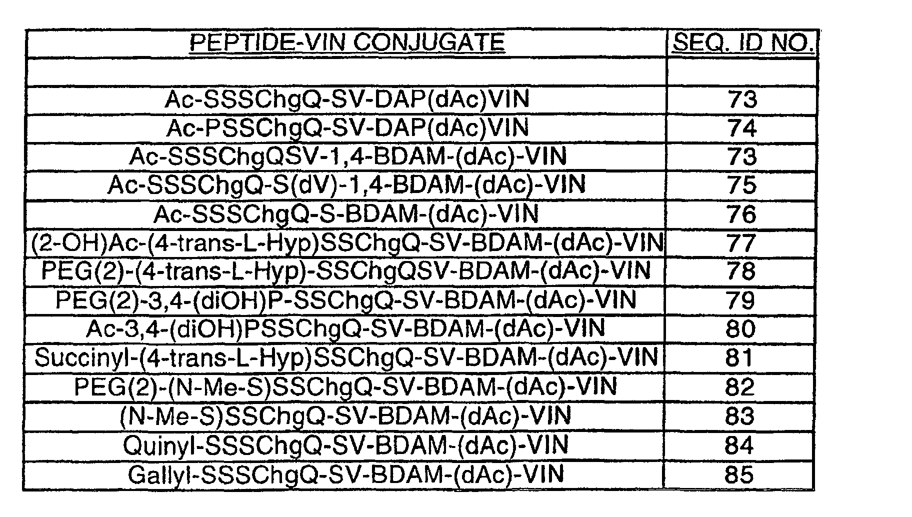 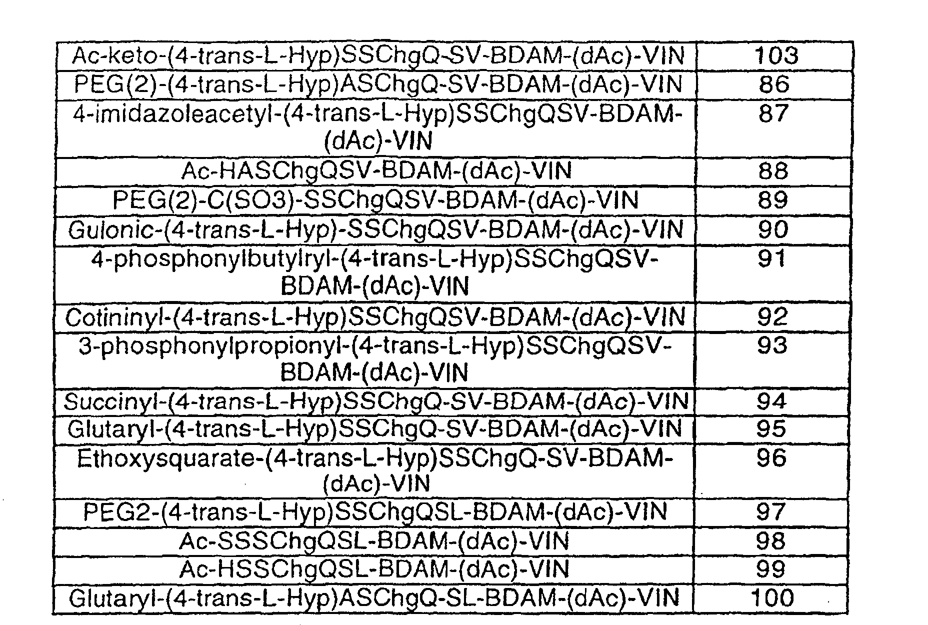 or a pharmaceutically acceptable salt or optical isomer thereof.

12. A pharmaceutical composition comprising a pharmaceutical carrier, and dispersed therein, a therapeutically effective amount of a compound of Claim 1, 6 or 11.

13. The use of a composition of Claim 12 for the manufacture of a medicament for treating prostate cancer.

14. The use of a composition of Claim 12 for the manufacture of a medicament for treating benign prostatic hyperplasia.

## Patentansprüche

1. Zur Behandlung von Prostatakrebs geeignetes Konjugat, welches ein zytotoxisches Vinca-Alkaloid-Agens in Verknüpfung mit einem Oligopeptid umfaßt, wobei das Oligopeptid eine Sequenz von Aminosäuren umfaßt, die von freiem prostataspezifischem Antigen selektiv proteolytisch gespalten wird, und wobei die Verknüpfung über einen chemischen Diamin-Linker erfolgt, welcher eine Cycloalkyloder Bicycloalkylgruppierung umfaßt,
oder das pharmazeutisch annehmbare Salz davon,
wobei das Oligopeptid nicht Semenogelin I, Semenogelin II, Fibronectin und IGFBP-3 einschließt.

2. Konjugat nach Anspruch 1, wobei das zytotoxische Agens aus den folgenden zytotoxischen Agentien:
a) Vinblastin,
b) 4-Desacetylvinbiastin,
c) Vincristin,
d) Leurosidin und
e) Vindesin,
ausgewählt ist;
oder das pharmazeutisch annehmbare Salz davon.

3. Konjugat nach Anspruch 2, wobei das zytotoxische Agens aus Vinblastin und 4-Desacetylvinblastin ausgewählt ist.

4. Konjugat nach Anspruch 1, wobei das Oligopeptid ein Oligomer umfaßt, welches ausgewählt ist aus:
a)
b)
c)
d)
e)
f)
g)
h)
i)
j)
k)
l)
m)
n)
o)
p)
q)
r)
s)
t)
u)
v)
w)
x)
y)
z)
wobei Haa eine cyclische Aminosäure ist, die mit einer hydrophilen Gruppierung substituiert ist, hArg Homoarginin ist, Xaa irgendeine Aminosäure ist, Cha Cyclohexylalanin ist und Chg Cyclohexylglycin ist.

5. Konjugat nach Anspruch 1, wobei das Oligopeptid ein Oligomer umfaßt, welches ausgewählt ist aus: und wobei 4-Hyp 4-Hydroxyprolin, 3,4-DiHyp 3,4-Dihydroxyprolin ist, N-Methyl-Ser Cyclohexylalanin ist und Chg Cyclohexylglycin ist.

6. Konjugat nach Anspruch 1 der Formel I: 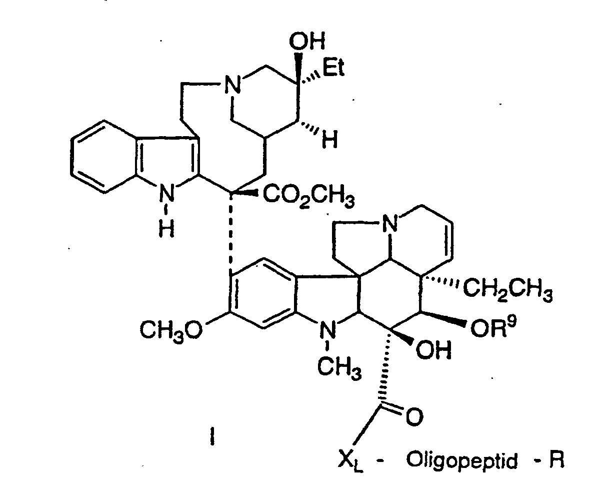 wobei:
das Oligopeptid ein Oligopeptid ist, welches spezifisch von dem freien prostataspezifischen Antigen (PSA) erkannt wird und in der Lage ist, von der enzymatischen Aktivität des freien prostataspezifischen Antigens proteolytisch gespalten zu werden,
X_{L} -NH-(CH₂)ᵤ-W-(CH₂)ᵤ-NH- ist,
R ausgewählt ist aus
a) Wasserstoff
b) -(C=O)R^{1a},
c) 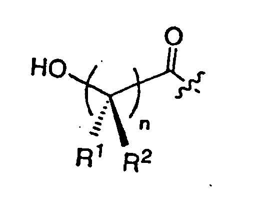
d) 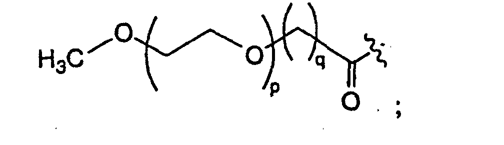
e) 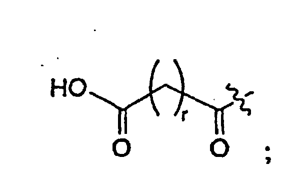
f) Ethoxysquarat und
g) Cotininyl;
R¹ und R² unabhängig aus Wasserstoff, OH, C₁-C₆-Alkyl, C₁-C₆-Alkoxy, C₁-C₆-Aralkyl und Aryl ausgewählt sind;
R^{1a} C₁-C₈-Alkyl oder Aryl ist;
R¹⁹ Wasserstoff, (C₁-C₃-Alkyl)-CO oder chlorsubstituiertes (C₁-C₃-Alkyl)-CO ist;
W aus Cyclopentyl, Cyclohexyl, Cycloheptyl oder Bicyclo[2.2.2]octanyl ausgewählt ist;
n 1, 2, 3 oder 4 ist;
p 0 oder eine ganze Zahl zwischen 1 und 100 ist;
q 0 oder 1 ist, vorausgesetzt, daß, wenn p 0 ist, q 1 ist;
r 1, 2 oder 3 ist;
t 3 oder 4 ist;
u 0, 1, 2 oder 3 ist;
oder ein pharmazeutisch annehmbares Salz davon.

7. Konjugat nach Anspruch 6, wobei das Oligopeptid ein Oligomer ist, welches eine Aminosäuresequenz umfaßt, die ausgewählt ist aus:
a)
b)
c)
d)
e)
f)
g)
h)
i)
j)
k)
l)
m)
n)
o)
p)
q)
r)
s)
t)
u)
v)
w)
x)
y)
z)
wobei Haa eine cyclische Aminosäure ist, die mit einer hydrophilen Gruppierung substituiert ist, hArg Homoarginin ist, Xaa irgendeine Aminosäure ist, Cha Cyclohexylalanin ist und Chg Cyclohexylglycin ist;
oder ein optisches Isomer oder pharmazeutisch annehmbares Salz davon.

8. Konjugat nach Anspruch 7, wobei:
Xaa Alanin, Serin oder Isoleucin ist, und
Haa *trans*-4-Hydroxy-L-Prolin ist;
oder ein optisches Isomer oder pharmazeutisch annehmbares Salz davon.

9. Konjugat nach Anspruch 8, welches ausgewählt ist aus: 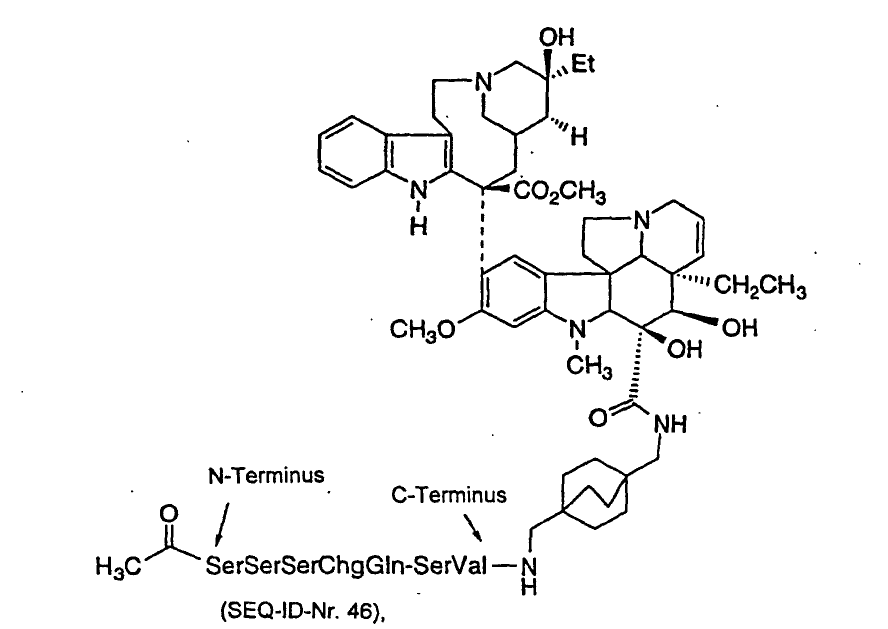 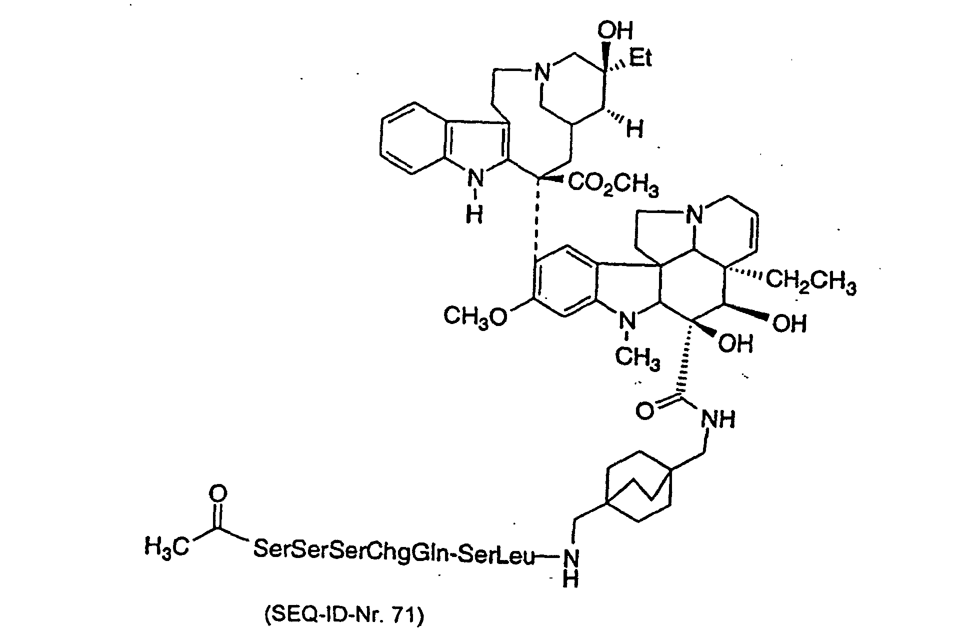 oder einem pharmazeutisch annehmbaren Salz oder optischen lsomer davon.

10. Konjugat nach Anspruch 8, welches ausgewählt ist aus: 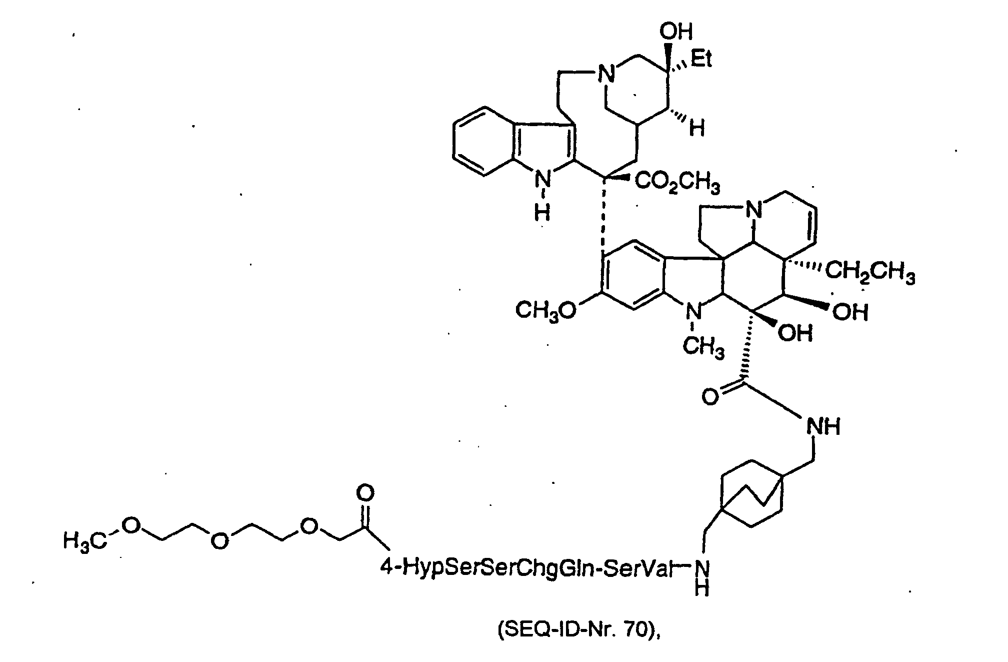 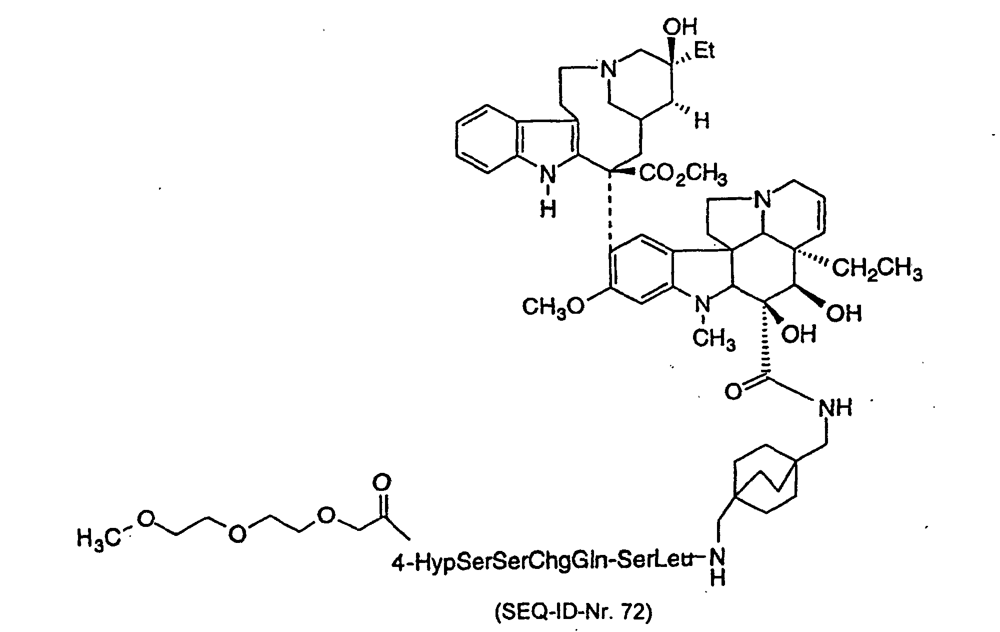 oder einem pharmazeutisch annehmbaren Salz oder optischen Isomer davon.

11. Verbindung, welche ausgewählt ist aus: 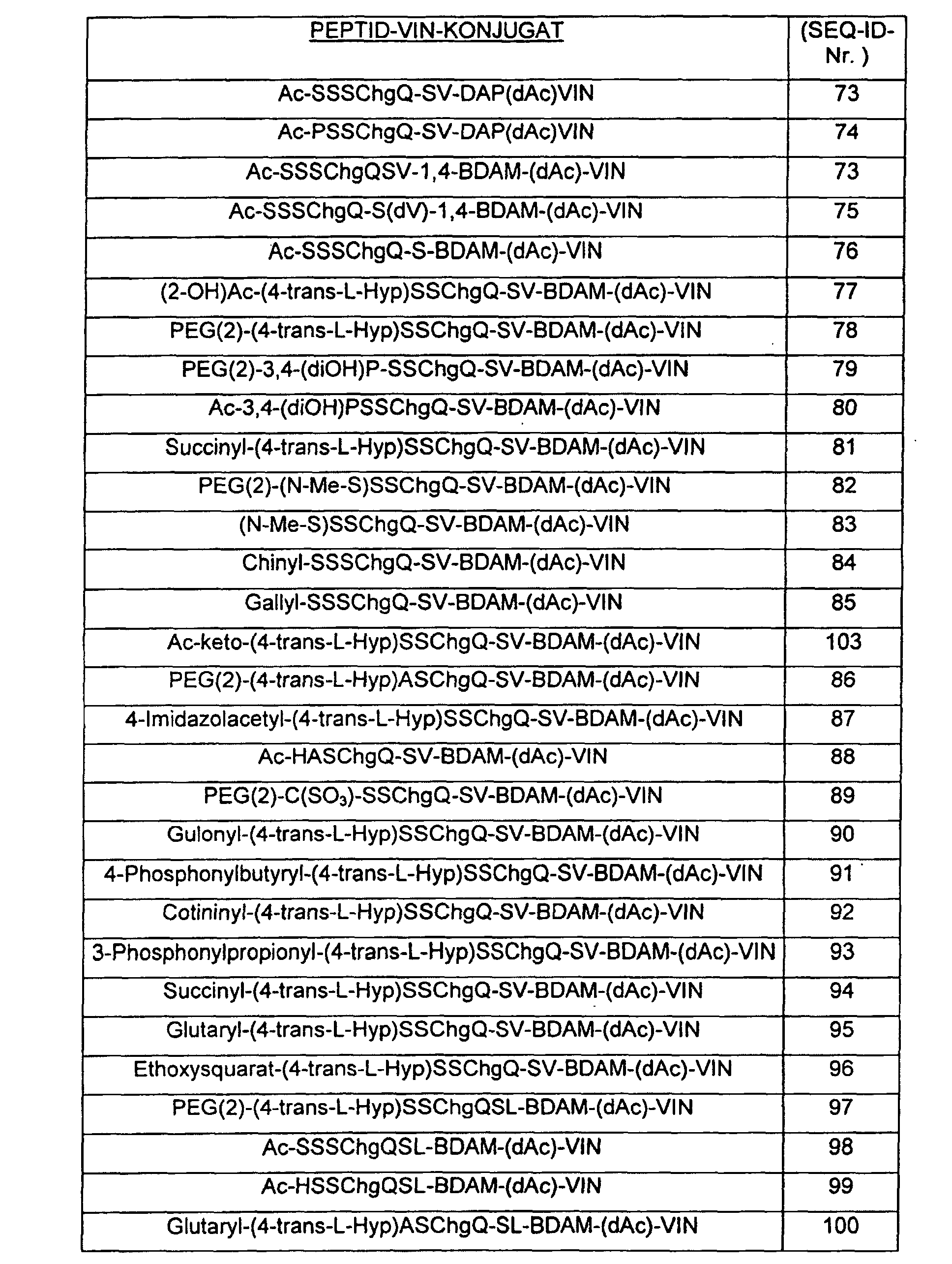 oder einem pharmazeutisch annehmbaren Salz oder optischen Isomer davon.

12. Pharmazeutische Zusammensetzung, umfassend einen pharmazeutischen Träger und darin dispergiert eine therapeutisch wirksame Menge einer Verbindung nach Anspruch 1, 6 oder 11.

13. Verwendung einer Zusammensetzung nach Anspruch 12 zur Herstellung eines Medikaments zur Behandlung von Prostatakrebs.

14. Verwendung einer Zusammensetzung nach Anspruch 12 zur Herstellung eines Medikaments zur Behandlung von gutartiger Prostata-Hyperplasie.

## Revendications

1. Conjugué qui est utile pour le traitement du cancer de la prostate qui comprend un agent cytotoxique de type alcaloïde de la pervenche attaché à un oligopeptide, dans lequel l'oligopeptide comprend une séquence d'acides aminés qui est sélectivement clivée par protéolyse par l'antigène prostatique spécifique libre et dans lequel le moyen d'attachement est par l'intermédiaire d'un lieur chimique de type diamine qui comprend un groupement cycloalkyle ou bicycloalkyle,
ou le sel pharmaceutiquement acceptable de celui-ci, où l'oligopeptide n'inclut pas la séménogéline I, la séménogéline II, la fibronectine et l'IGFβR-3.

2. Conjuqué selon la revendication 1 dans lequel l'agent cytotoxique est choisi parmi les agents cytotoxiques suivants :
a) vinblastine,
b) 4-désacétylvinblastine,
c) vincristine,
d) leurosidine, et
e) vindésine,
ou le sel pharmaceutiquement acceptable de celui-ci.

3. Conjugué selon la revendication 2 dans lequel l'agent cytotoxique est choisi parmi la vinblastine et la 4-désacétylvinblastine.

4. Conjugué selon la revendication 1 dans lequel l'oligopeptide comprend un oligomère choisi parmi :
a)
b)
c)
d)
e)
f)
g)
h)
i)
j)
k)
l)
m)
n)
o)
p)
q)
r)
s)
t)
u)
v)
w)
x)
y)
z)
où Haa est un acide aminé cyclique substitué par un groupement hydrophile, hArg est une homoarginine, Xaa est un acide aminé quelconque, Cha est une cyclohexylalanine et Chg est une cyclohexylglycine.

5. Conjugué selon la revendication 1 dans lequel l'oligopeptide comprend un oligomère choisi parmi : et où 4-Hyp est une 4-hydroxyproline, 3,4-DiHyp est une 3,4-dihydroxyproline, N-méthyl-Ser est une cyclohexylalanine et Chg est une cyclohexylglycine.

6. Conjugué selon la revendication 1 de formule I : 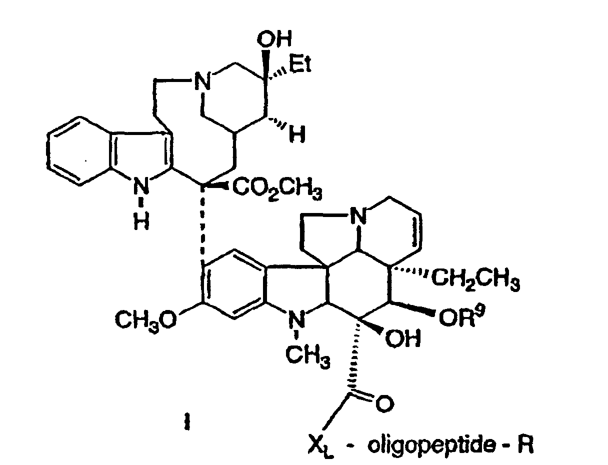 dans laquelle :
oligopeptide est un oligopeptide qui est spécifiquement reconnu par l'antigène prostatique spécifique (PSA) libre et est capable d'être clivé par protéolyse par l'activité enzymatique de l'antigène prostatique spécifique libre,
X_{L} est -NH-(CH₂)ᵤ-W-(CH₂)ᵤ-NH-
R est choisi parmi
a) un hydrogène,
b) -(C=O)R^{1a},
c) 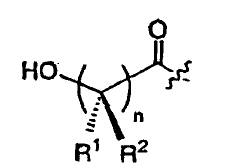
d) 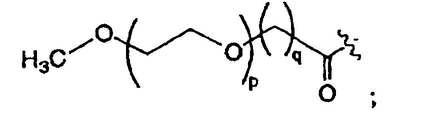
e) 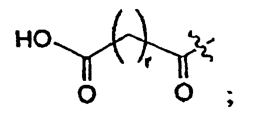
f) un groupe éthoxysquarate ; et
g) un groupe cotininyle ;
R¹ et R² sont indépendamment choisis parmi : un hydrogène, OH, un groupe alkyle en C₁-C₆, alcoxy en C₁-C₆, aralkyle en C₁-C₆ et aryle ;
R^{1a} est un groupe alkyle en C₁-C₆ ou aryle ;
R¹⁹ est un hydrogène, un groupe (alkyle en C₁-C₃)-CO, ou un groupe (alkyle en C₁-C₃)-CO chlorosubstitué ;
W est choisi parmi un groupe cyclopentyle, cyclohexyle, cycloheptyle ou bicyclo[2.2.2]octanyle ;
n vaut 1, 2, 3 ou 4 ;
p vaut zéro ou est un entier entre 1 et 100 ;
q vaut 0 ou 1, sous réserve que si p vaut zéro, q vaut 1 ;
r vaut 1, 2 ou 3 ;
t vaut 3 ou 4 ;
u vaut 0, 1, 2 ou 3,
ou un de ses sels pharmaceutiquement acceptables.

7. Conjugué selon la revendication 6 dans lequel :
oligopeptide est un oligomère qui comprend une séquence d'acides aminés choisie parmi :
a)
b)
c)
d)
e)
f)
g)
h)
i)
j)
k)
l)
m)
n)
o)
p)
q)
r)
s)
t)
u)
v)
w)
x)
y)
z)
où Haa est un acide aminé cyclique substitué par un groupement hydrophile, hArg est une homoarginine, Xaa est un acide aminé quelconque, Cha est une cyclohexylalanine et Chg est une cyclohexylglycine ;
ou un isomère optique ou sel pharmaceutiquement acceptable de celui-ci.

8. Conjugué selon la revendication 7 dans lequel :
Xaa est une alanine, sérine ou isoleucine ; et
Haa est une *trans*-4-hydroxy-L-proline ;
ou un isomère optique ou sel pharmaceutiquement acceptable de celui-ci.

9. Conjugué selon la revendication 8 qui est choisi parmi : 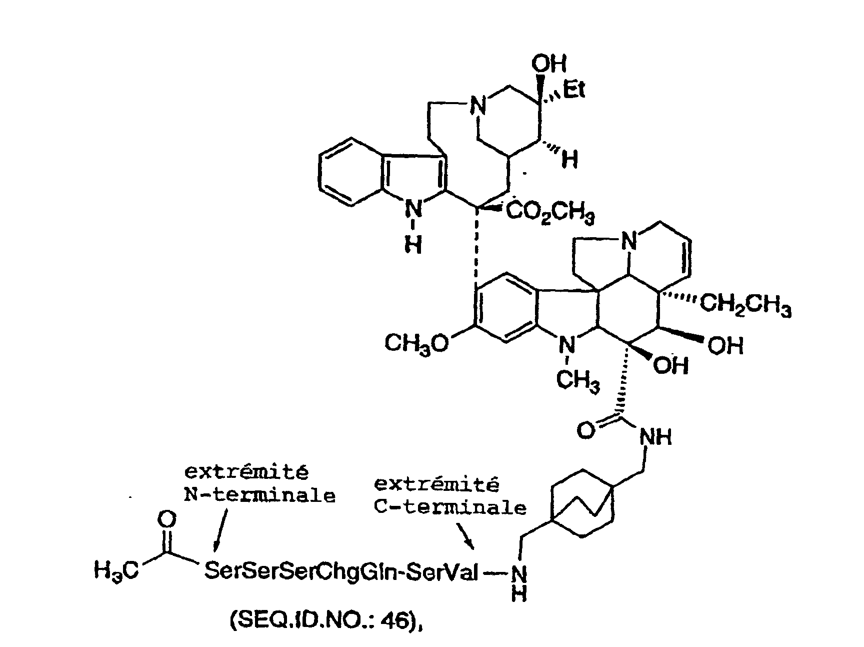 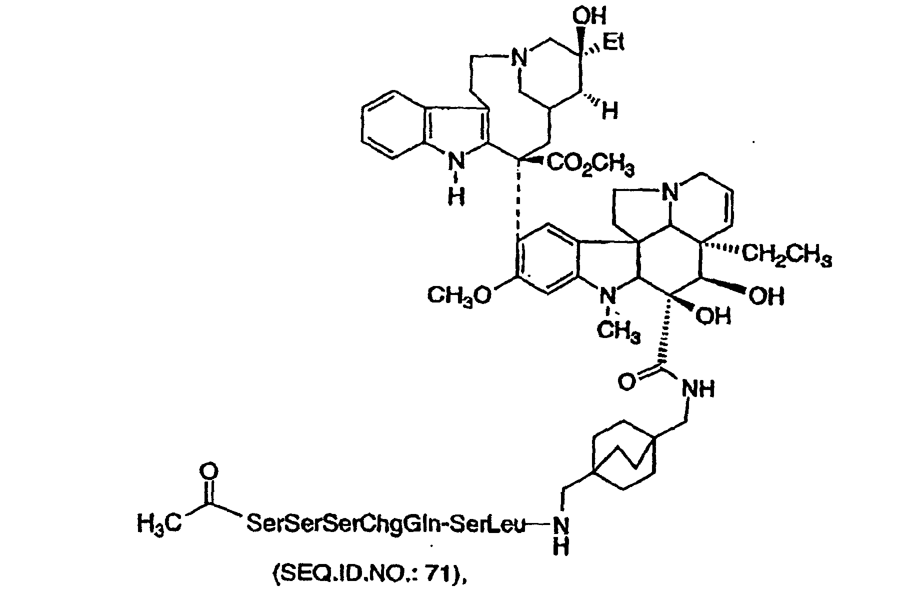 ou un isomère optique ou sel pharmaceutiquement acceptable de celui-ci.

10. Conjugué selon la revendication 8 qui est choisi parmi : 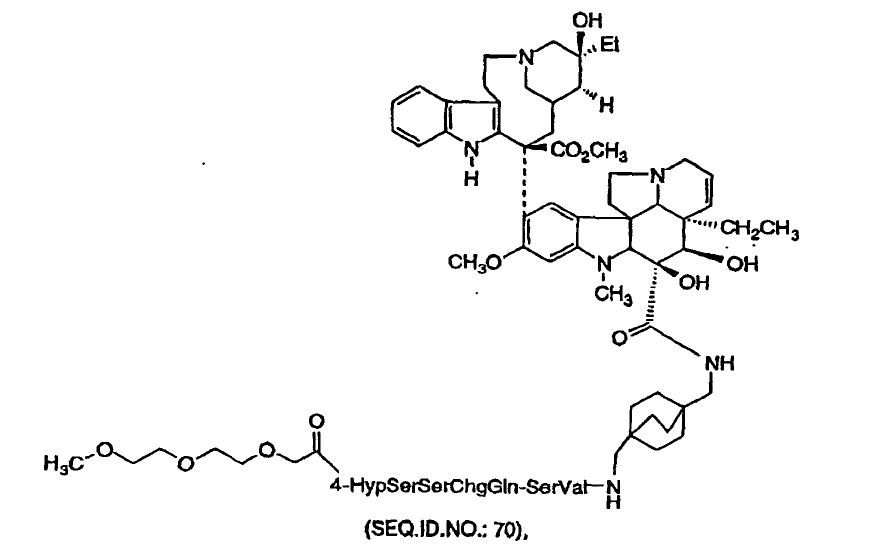 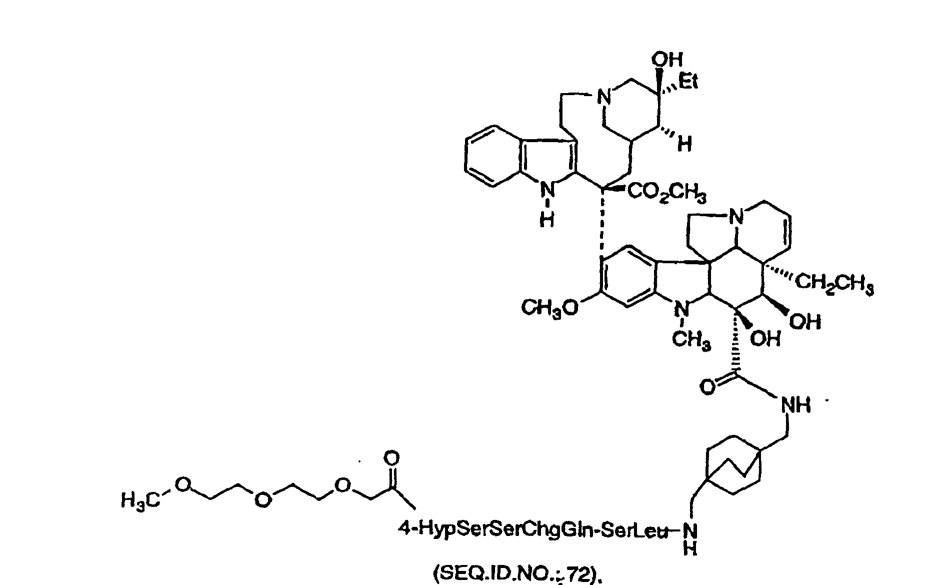 ou un isomère optique ou sel pharmaceutiquement acceptable de celui-ci.

11. Composé qui est choisi parmi : 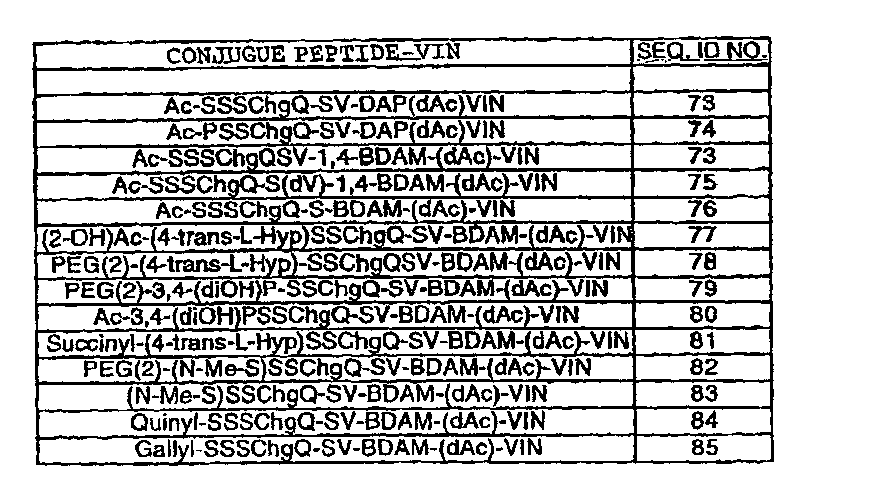 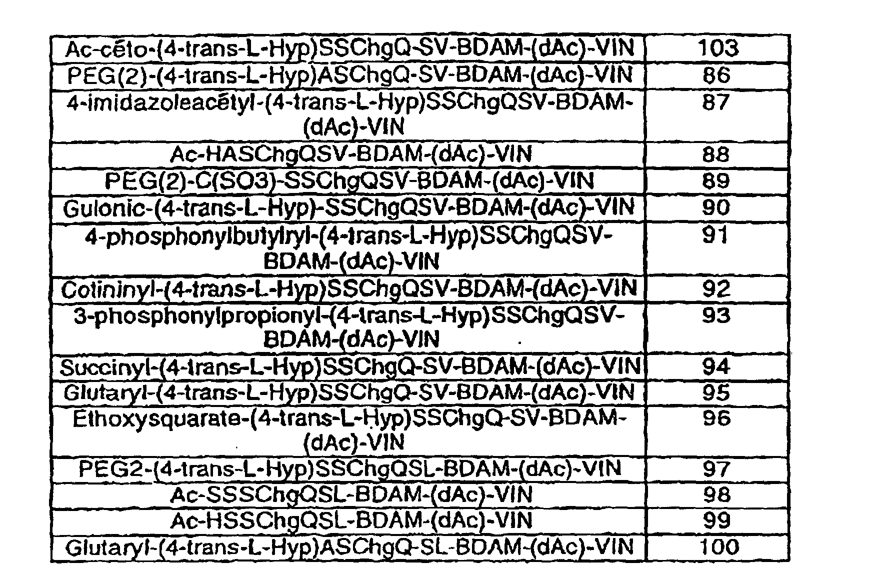 ou un isomère optique ou sel pharmaceutiquement acceptable de celui-ci.

12. Composition pharmaceutique comprenant un support pharmaceutique, et dispersée dans ce dernier, une quantité thérapeutiquement efficace d'un composé selon la revendication 1, 6 ou 11.

13. Utilisation d'une composition selon la revendication 12 pour la fabrication d'un médicament destiné au traitement du cancer de la prostate.

14. utilisation d'une composition selon la revendication 12 pour la fabrication d'un médicament destiné au traitement de l'hyperplasie prostatique bénigne.
